(19) Europäisches Patentamt
European Patent Office
Office européen des brevets

(11) EP 4 365 279 A1

(12) # EUROPEAN PATENT APPLICATION
published in accordance with Art. 153(4) EPC

(43) Date of publication:
**08.05.2024 Bulletin 2024/19**

(21) Application number: **22833084.1**

(22) Date of filing: **27.06.2022**

(51) International Patent Classification (IPC):
***C12N 1/02*** *(2006.01)* ***C12N 5/071*** *(2010.01)*
***G01N 33/53*** *(2006.01)* ***G01N 33/543*** *(2006.01)*

(52) Cooperative Patent Classification (CPC):
**C12N 1/02; C12N 5/06; G01N 33/53; G01N 33/543**

(86) International application number:
**PCT/JP2022/025542**

(87) International publication number:
**WO 2023/276937 (05.01.2023 Gazette 2023/01)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **30.06.2021 JP 2021109278**

(71) Applicants:
- **NOF Corporation**
**Shibuya-ku**
**Tokyo 150-6019 (JP)**
- **Tokyo Institute of Technology**
**Tokyo 152-8550 (JP)**

(72) Inventors:
- **IMAMURA, Ryutaro**
**Kawasaki-shi, Kanagawa 210-0821 (JP)**
- **OHTAKE, Tomoyuki**
**Kawasaki-shi, Kanagawa 210-0821 (JP)**
- **MARUYAMA, Atsushi**
**Tokyo 152-8550 (JP)**
- **SHIMADA, Naohiko**
**Tokyo 152-8550 (JP)**

(74) Representative: **J A Kemp LLP**
**80 Turnmill Street**
**London EC1M 5QU (GB)**

(54) **CELL DISSOCIATING AGENT AND CELL SEPARATION METHOD**

(57) The present invention provides a cell detachment agent for detaching a cell to be separated from a separation carrier bound to the cell to be separated, wherein the above-mentioned cell detachment agent contains a bindable ureido group-containing polymer having a side chain structure represented by the following formula (1) and having a separation carrier-binding site capable of binding to the above-mentioned separation carrier at a side chain or terminal thereof, and the above-mentioned bindable ureido group-containing polymer has an upper critical solution temperature (UCST) of 4 to 40°C:

$$-NH-(C=O)-NH_2 \cdots \quad (1)$$

EP 4 365 279 A1

## Description

[Technical Field]

The present invention relates to cell detachment agents for use in cell separation methods using surface antigens of cells to be separated, and the like.

[Background Art]

In the field of regenerative medicine, a method of extracting normal cells from a patient with a disease, culturing them ex-vivo, and then transplanting them back into the patient is often used. However, it is difficult to extract only specific cells from the patient's body, blood, and body fluids. Therefore, a technique for separating specific cells is required.

This cell separation method is also spreading in the fields of pathological diagnosis and clinical testing. As cell separation methods, density gradient centrifugation method, separation methods using magnetic particles, separation methods using substrates, and methods combining flow cytometry and cell sorter are known. Among these, separation methods using magnetic particles are methods that can process a large number of cells and afford cells with high purity after separation.

In general cell separation methods using magnetic particles, a method that utilizes the desthiobiotin-streptavidin interaction is generally performed. In this method, first, an antibody specific to the cells to be separated is desthiobiotinylated. The desthiobiotinylated antibody is reacted with the surface antigen of the cells to be separated, and the cells are captured using magnetic particles having streptavidin on the surface thereof. The magnetic particles are collected by magnetic force to separate the cells to be separated from a cell population including multiple cell types, after which a large amount of biotin is added as a cell detachment agent to allow competition with the desthiobiotin-streptavidin interaction, thus detaching the cells to be separated and magnetic particles. However, since the method requires detachment of the cells from the separation carrier by using a large amount of biotin, it is necessary to wash the separated cells multiple times, which poses a problem that the operation requires a long time.

As a cell separation method that does not use biotin, Patent Literature 1 discloses a method in which a separation carrier and cells are bonded using an enzymatically degradable linker such as dextran, and the cells are detached from the separation carrier by cleaving the linker using an enzyme. However, this cell separation method is also associated with a problem that the operation requires a long time because the separated cells need to be washed multiple times due to a risk that the enzyme used to degrade the linker may damage the separated cells.

As a cell separation method that does not use a large amount of biotin or enzyme, Patent Literature 2 discloses a method for specifically separating T cells that uses strept-tagged MHC multimers. In this method, strept-tagged MHC multimers bound to surface antigen of T cells are bound to magnetic particles containing streptotactin, and cells to be separated are sorted. Thereafter, the cells to be separated are detached using a small amount of biotin as a cell detachment agent. Even though this cell separation method causes little damage to cells, the strept-tagged MHC multimer used is applicable only to T cells and cannot be applied to other types of cells, which poses a problem of low versatility.

[Citation List]

[Patent Literature]

[PTL 1]
JP-A-2016-136937
[PTL 2]
JP-A-2017-514481

[Summary of Invention]

[Technical Problem]

As described above, a cell detachment agent is not known that can be applied to broad types of cells in a cell separation method using surface antigen of cells to be separated, and that does not require washing after separation of the cells to be separated.

An object of the present invention is to provide a cell detachment agent that can be applied to broad types of cells in a cell separation method using surface antigen of cells to be separated, and that does not require washing after

separation of the cells to be separated, and a cell separation method that uses the cell detachment agent.

[Solution to Problem]

The present inventors have conducted intensive studies in an attempt to achieve the aforementioned object and found that the expansion and/or contraction of polymer chains due to temperature response can be used as a driving force to detach cells to be separated from a separation carrier, and that a cell detachment agent containing a polymer that does not affect the cells after separation can solve the above-mentioned problem.

That is, the present invention provides at least the following [1] to [22].

[1] A cell detachment agent for detaching a cell to be separated from a separation carrier bound to the cell to be separated, wherein

the above-mentioned cell detachment agent comprises a bindable ureido group-containing polymer having a side chain structure represented by the following formula (1) and having a separation carrier-binding site capable of binding to the above-mentioned separation carrier at a side chain or terminal thereof, and
the above-mentioned bindable ureido group-containing polymer has an upper critical solution temperature (UCST) of 4 to 40°C:

$$-NH-(C=O)-NH_2 \cdots \quad (1).$$

[2] The cell detachment agent of the above-mentioned [1], wherein the above-mentioned separation carrier-binding site is a biotin residue, a streptavidin residue, an amino group, a carboxyl group, a mercapto group, a cyclodextrin residue, an adamantyl group, or a phenylboronic acid residue.
[3] The cell detachment agent of the above-mentioned [1] or [2], wherein the above-mentioned ureido group-containing polymer has a main chain which is an ethylenically-unsaturated polymer, a polyallylamine, or a polypeptide.
[4] The cell detachment agent of any of the above-mentioned [1] to [3], wherein the above-mentioned ureido group-containing polymer comprises a ureido group-containing repeating sequence A that is a repeat of a ureido group-containing structure represented by the following formula (2):

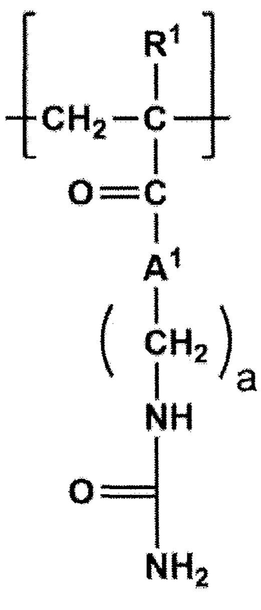
· · · (2)

in the above-mentioned formula (2), $R^1$ is a hydrogen atom or a methyl group, $A^1$ is O or NH, and a is an integer of 1 to 6.
[5] The cell detachment agent of the above-mentioned [4], wherein the above-mentioned ureido group-containing polymer further comprises a hydrophilic repeating sequence B that is a repeat of a hydrophilic structure represented by the following formula (3):

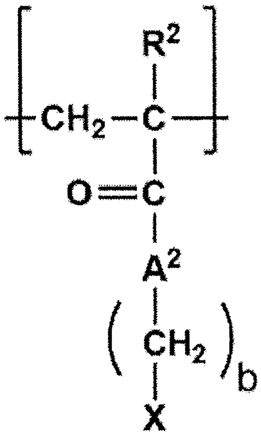

• • • (3)

in the above-mentioned formula (3), $R^2$ is a hydrogen atom or a methyl group, $A^2$ is O or NH, b is an integer of 1 to 6, and X is selected from the following formulas (4) to (9):

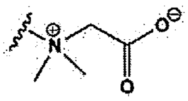 • • • (7) 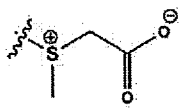 • • • (8) 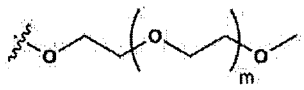 • • • (9)

in the above-mentioned formula (9), m is an integer of 0 to 20.

[6] The cell detachment agent of the above-mentioned [5], wherein, in the above-mentioned ureido group-containing repeating sequence A, $A^1$ is O and a is 2, and in the above-mentioned hydrophilic repeating sequence B, $A^2$ is O, b is 2, and X is represented by the above-mentioned formula (4).

[7] The cell detachment agent of the above-mentioned [5] or [6], wherein the above-mentioned ureido group-containing repeating sequence A and the above-mentioned hydrophilic repeating sequence B in the above-mentioned ureido group-containing polymer are in a relationship of $0.5 \leq A/(A+B)$ in a molar ratio.

[8] The cell detachment agent of any of the above-mentioned [4] to [7], wherein the above-mentioned bindable ureido group-containing polymer has the biotin residue as the above-mentioned separation carrier-binding site at the terminal of the above-mentioned ureido group-containing polymer.

[9] The cell detachment agent of any of the above-mentioned [1] to [3], wherein the above-mentioned ureido group-containing polymer comprises a ureido group-containing repeating sequence C that is a repeat of a ureido group-containing structure represented by the following formula (10) and a hydrophilic repeating sequence D that is a repeat of a hydrophilic structure represented by the following formula (11):

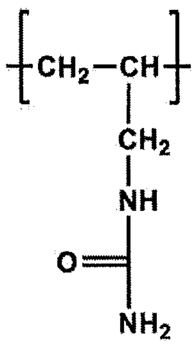 • • • (10) 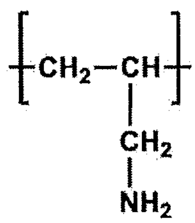 • • • (11).

[10] The cell detachment agent of the above-mentioned [9], wherein the above-mentioned ureido group-containing repeating sequence C and the above-mentioned hydrophilic repeating sequence D in the above-mentioned ureido group-containing polymer are in a relationship of $0.5 \leq C/(C+D)$ in a molar ratio.

[11] The cell detachment agent of the above-mentioned [9] or [10], wherein the agent comprises the above-mentioned bindable ureido group-containing polymer having the biotin residue as the above-mentioned separation carrier-binding site at the side chain of the above-mentioned ureido group-containing polymer.

[12] The cell detachment agent of any of the above-mentioned [1] to [3], wherein the above-mentioned ureido group-containing polymer comprises a ureido group-containing repeating sequence E that is a repeat of a ureido group-containing structure represented by the following formula (12) and a hydrophilic repeating sequence F that is a repeat of a hydrophilic structure represented by the following formula (13):

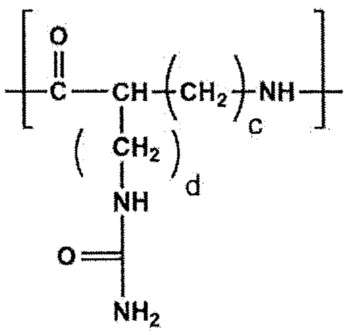
· · · (12)
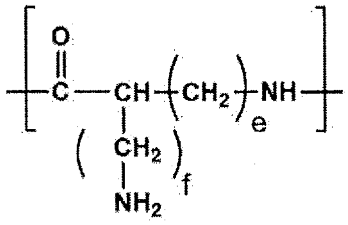
· · · (13)

in the above-mentioned formula (12), c is an integer of 0 to 6 and d is an integer of 0 to 6, and in the above-mentioned formula (13), e is an integer of 0 to 6 and f is an integer of 0 to 6.

[13] The cell detachment agent of the above-mentioned [12], wherein, in the above-mentioned ureido group-containing repeating sequence E, c is 0 and d is 3, and in the above-mentioned hydrophilic repeating sequence F, e is 0 and f is 3.

[14] The cell detachment agent of the above-mentioned [12] or [13], wherein the above-mentioned ureido group-containing repeating sequence E and the above-mentioned hydrophilic repeating sequence F in the above-mentioned ureido group-containing polymer are in a relationship of $0.5 \leq E/(E+F)$ in a molar ratio.

[15] The cell detachment agent of any of the above-mentioned [12] to [14], wherein the agent comprises the above-mentioned bindable ureido group-containing polymer having the biotin residue as the above-mentioned separation carrier-binding site at the side chain of the above-mentioned ureido group-containing polymer.

[16] The cell detachment agent of any of the above-mentioned [12] to [15], wherein the above-mentioned ureido group-containing polymer has a number average molecular weight of 20,000 to 1,000,000.

[17] A method for separating a cell to be separated from a cell population comprising the cell to be separated, comprising the following [step A], [step B], [step C], and [step D]:

[step A]
a step of adding a substance having a separation carrier-binding site and recognizing a surface antigen of the cell to be separated to a cell population comprising the cell to be separated to cause a reaction with the surface antigen of the cell to be separated, thus obtaining a complex of the cell to be separated-substance recognizing the antigen
[step B]
a step of interacting the complex of the cell to be separated-substance recognizing the antigen obtained in [step A] with a separation carrier via a separation carrier-binding site to obtain a complex of the cell to be separated-separation carrier
[step C]
a step of separating only the complex of the cell to be separated-separation carrier obtained in [step B] from the cell population comprising the cell to be separated
[step D]
a step of adding a cell detachment agent of any of the above-mentioned [1] to [16] to the complex of the cell to be separated-separation carrier obtained in [step C] and separating the cell to be separated by temperature change.

[18] The cell separation method of the above-mentioned [17], wherein the separation carrier-binding site in the above-mentioned [step A] is a biotin residue, a streptavidin residue, an amino group, a carboxyl group, a mercapto group, a cyclodextrin residue, an adamantyl group, or a phenylboronic acid residue.

[19] The cell separation method of the above-mentioned [17] or [18], wherein the substance recognizing the antigen in the above-mentioned [step A] is an antibody, an antibody fragment, a peptide, an aptamer, protein A, or lectin.

[20] The cell separation method of any of the above-mentioned [17] to [19], wherein the separation carrier in the above-mentioned [step B] is a magnetic particle.

[21] The cell separation method of the above-mentioned [20], wherein only the complex of the cell to be separated-separation carrier is separated by magnetic force from the cell population comprising the cell to be separated in the above-mentioned [step C].

[22] The cell separation method of any of the above-mentioned [17] to [21], wherein the temperature change in the above-mentioned [step D] comprises heating from a temperature not more than the upper critical solution temperature (UCST) to a temperature not less than the upper critical solution temperature (UCST) of the bindable ureido group-containing polymer.

[Advantageous Effects of Invention]

According to the present invention, a cell detachment agent that can be applied to a wide range of cell types and does not require washing after separation of the cells to be separated, in a cell separation method using a surface antigen of cells to be separated, and a cell separation method using the cell detachment agent can be provided.

[Description of Embodiments]

Preferred embodiments of the present invention are described in detail below.

<Ureido group-containing polymer>

The above-mentioned ureido group-containing polymer refers to a polymer having a ureido group represented by the following formula (1) (hereinafter referred to as ureido group) in the side chain:

$$-NH-(C=O)-NH_2 \cdots \quad (1).$$

The upper critical solution temperature (UCST) of the above-mentioned ureido group-containing polymer is basically almost the same as the upper critical solution temperature (UCST) of a bindable ureido group-containing polymer having, at the side chain or terminal thereof, a separation carrier-binding site capable of binding to a separation carrier, as described below, and is preferably within the range of 4 to 40°C.

The main chain of the above-mentioned ureido group-containing polymer is not particularly limited, and an ethylenically-unsaturated polymer, polyallylamine, polypeptide, polyester, polysaccharide, and the like can be mentioned. Among these, an ethylenically-unsaturated polymer, polyallylamine, or polypeptide is preferred since synthesis is easy.

When the main chain of the above-mentioned ureido group-containing polymer is an ethylenically-unsaturated polymer, a structure having ureido group-containing repeating sequence A that is a repeat of a ureido group-containing structure represented by the following formula (2) is preferred

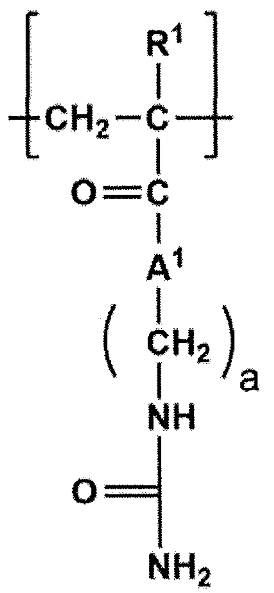

・・・(2)

in the above-mentioned formula (2), $R^1$ is a hydrogen atom or a methyl group, $A^1$ is O or NH, and a is an integer of 1 to 6.

The "repeats of the structure" as used in the present specification refers to continuous repeats of the structure,

as well as intermittent repeats.

In the above-mentioned formula (2), a is an integer of 1 to 6, preferably an integer of 1 to 4, most preferably an integer of 2 or 3.

$R^1$, $A^1$, and a in the respective ureido group-containing structures represented by the formula (2) in the above-mentioned ureido group-containing repeating sequences A may be the same or different.

Examples of the above-mentioned ureido group-containing repeating sequence A include a ureido group-containing repeating sequence A wherein $R^1$ is a methyl group, $A^1$ is O, and a is 2, a ureido group-containing repeating sequence A wherein $R^1$ is a methyl group, $A^1$ is NH, and a is 2, a ureido group-containing repeating sequence A wherein $R^1$ is a hydrogen atom, $A^1$ is O, and a is 2, a ureido group-containing repeating sequence A wherein $R^1$ is a hydrogen atom, $A^1$ is NH, and a is 2, and the like. From the aspect of copolymerizability with the below-mentioned other monomers, a ureido group-containing repeating sequence A wherein $R^1$ is a methyl group, $A^1$ is O, and a is 2, a ureido group-containing repeating sequence A wherein $R^1$ is a hydrogen atom, $A^1$ is O, and a is 2, or a ureido group-containing repeating sequence A wherein $R^1$ is a hydrogen atom, $A^1$ is NH, and a is 2 is preferred, and a ureido group-containing repeating sequence A wherein $R^1$ is a methyl group, $A^1$ is O, and a is 2 is more preferred.

When the main chain of the above-mentioned ureido group-containing polymer is an ethylenically-unsaturated polymer, the above-mentioned ureido group-containing polymer may be a copolymer of a monomer having a ureido group-containing structure such as a ureido group-containing structure represented by the above-mentioned formula (2) and the like and, for example, the following other monomer, and the above-mentioned upper critical solution temperature (UCST) can be adjusted by copolymerization with other monomers. In order to lower the upper critical solution temperature (UCST), for example, hydrophilic monomers can be selected as other monomers and, for example, glycerol (meth)acrylate, (meth)acryloyloxyethyl phosphate, N-methylcarboxybetaine (meth)acrylate, N-methylsulfobetaine (meth)acrylate, aminoethyl (meth)acrylate, N,N'-dimethylacrylamide, S-methylsulfonium carboxylic acid (meth)acrylate, ethylene glycol (meth)acrylate, and the like can be mentioned. When a ureido group-containing polymer having an ethylenically-unsaturated polymer at least as the above-mentioned main chain is used as the starting material of the cell detachment agent, (meth)acryloyloxyethyl phosphate, N-methylcarboxybetaine (meth)acrylate, N-methylsulfobetaine (meth)acrylate, aminoethyl (meth)acrylate, S-methylsulfonium carboxylic acid (meth)acrylate, or ethylene glycol (meth)acrylate is preferably used from the aspects of solubility in water, suppression of nonspecific adsorption, and the like. More specifically, a structure having a hydrophilic repeating sequence B that is a repeat of a hydrophilic structure represented by the following formula (3) in addition to the above-mentioned ureido group-containing repeating sequence A is preferred.

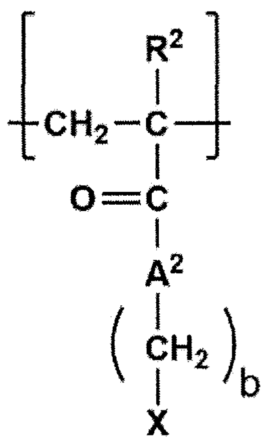

• • • (3)

in the above-mentioned formula (3), $R^2$ is a hydrogen atom or a methyl group, $A^2$ is O or NH, b is an integer of 1 to 6, and X is selected from the following formulas (4) to (9): in the above-mentioned formula (9), m is an integer of 0 to 20.

In the above-mentioned formula (3), b is an integer of 1 to 6, preferably an integer of 1 to 4, most preferably an integer of 2 or 3.

$R^2$, $A^2$, and b in respective hydrophilic structures represented by the formula (3) in the above-mentioned hydrophilic repeating sequences B may be the same or different.

Examples of the above-mentioned hydrophilic repeating sequence B include a ureido group-containing repeating sequence B wherein $R^2$ is a methyl group, $A^2$ is O, b is 2, and X is selected from the above-mentioned formulas (4) to (9), a ureido group-containing repeating sequence B wherein $R^2$ is a methyl group, $A^2$ is NH, b is 2, and X is selected from the above-mentioned formulas (4) to (9), a ureido group-containing repeating sequence B wherein $R^2$ is a hydrogen atom, $A^2$ is O, b is 2, and X is selected from the above-mentioned formulas (4) to (9), a ureido group-containing repeating

sequence B wherein $R^2$ is a hydrogen atom, $A^2$ is NH, b is 2, and X is the above-mentioned formulas (4) to (9), and the like. From the aspect of copolymerization with monomer having a ureido group-containing structure represented by the above-mentioned formula (2), and the like, a ureido group-containing repeating sequence B wherein $R^2$ is a methyl group, $A^2$ is O, b is 2, and X is selected from the above-mentioned formulas (4) to (9), a ureido group-containing repeating sequence B wherein $R^2$ is a hydrogen atom, $A^2$ is O, b is 2, and X is selected from the above-mentioned formulas (4) to (9), or a ureido group-containing repeating sequence B wherein $R^2$ is a hydrogen atom, $A^2$ is NH, b is 2, and X is the above-mentioned formulas (4) to (9) is preferred, and a ureido group-containing repeating sequence B wherein $R^2$ is a methyl group, $A^2$ is 0, b is 2, and X is selected from the above-mentioned formulas (4) to (9) is most preferred.

When the above-mentioned ureido group-containing polymer with an ethylenically-unsaturated polymer as the main chain is a copolymer with the above-mentioned other monomer, the molar ratio of the above-mentioned other monomer can be appropriately determined such that the required function may be exhibited. In order to set the upper critical solution temperature of the above-mentioned bindable ureido group-containing polymer to 4 to 40°C, the content of the above-mentioned other monomer to be blended is preferably not more than 70 mol%, more preferably not more than 60 mol%, further preferably not more than 50 mol%, further more preferably not more than 40 mol%, particularly preferably not more than 30 mol%, particularly more preferably not more than 20 mol%, of the whole monomer. That is, ureido group-containing repeating sequences such as the above-mentioned ureido group-containing repeating sequence A and the like are preferably not less than 30 mol%, more preferably not less than 40 mol%, further preferably not less than 50 mol%, further more preferably not less than 60 mol%, particularly preferably not less than 70 mol%, particularly more preferably not less than 80 mol%, of the whole sequences. When the above-mentioned ureido group-containing polymer has a structure including the above-mentioned ureido group-containing repeating sequence A and the above-mentioned hydrophilic repeating sequence B, a molar ratio (number of moles) of the above-mentioned ureido group-containing repeating sequence A and the above-mentioned hydrophilic repeating sequence B is preferably within the range of $0.3 \leq A/(A+B)$. More preferably the relationship of $0.4 \leq A/(A+B)$ is satisfied, more preferably the relationship of $0.5 \leq A/(A+B)$ is satisfied, further more preferably the relationship of $0.6 \leq A/(A+B)$ is satisfied, particularly preferably the relationship of $0.7 \leq A/(A+B)$ is satisfied, particularly more preferably the relationship of $0.8 \leq A/(A+B)$ is satisfied.

The above-mentioned ureido group-containing polymer having an ethylenically-unsaturated polymer as the main chain is not particularly limited as long as it has a ureido group and has an upper critical solution temperature (UCST) in the range of 4 to 40°C. The upper critical solution temperature (UCST) can be adjusted according to the combination of the type of the main chain type, the type of other monomers, and molecular weight, and the like.

When the main chain of the above-mentioned ureido group-containing polymer is polyallylamine, a structure having a ureido group-containing repeating sequence C that is a repeat of a ureido group-containing structure represented by the following formula (10) and a hydrophilic repeating sequence D that is a repeat of a hydrophilic structure represented by the following formula (11) is preferred:

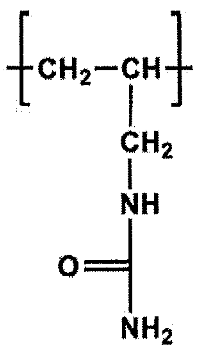 · · · (10)

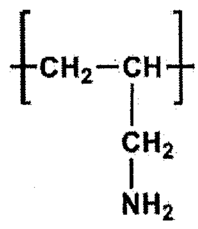 · · · (11).

In order to set the upper critical solution temperature of the above-mentioned ureido group-containing polymer with polyallylamine as the main chain to 4 to 40°C, ureido group-containing repeating sequences such as the above-mentioned ureido group-containing repeating sequence C and the like are preferably not less than 30 mol%, more preferably not less than 40 mol%, further preferably not less than 50 mol%, of the whole sequences.

When the above-mentioned ureido group-containing polymer has a structure including the above-mentioned ureido group-containing repeating sequence C and the above-mentioned hydrophilic repeating sequence D, a molar ratio (number of moles) of the above-mentioned ureido group-containing repeating sequence C and the above-mentioned hydrophilic repeating sequence D is preferably within the range of $0.3 \leq C/(C+D)$. More preferably the relationship of $0.4 \leq C/(C+D)$ is satisfied, and particularly preferably, the relationship of $0.5 \leq C/(C+D)$ is satisfied.

When the main chain of the above-mentioned ureido group-containing polymer is polypeptide, a structure including a ureido group-containing repeating sequence E that is a repeat of a ureido group-containing structure represented by the following formula (12) and a hydrophilic repeating sequence F that is a repeat of a hydrophilic structure represented by the following formula (13) is preferred:

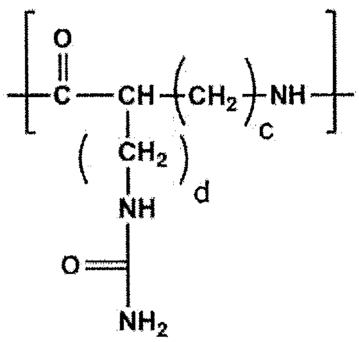
・・・(12)

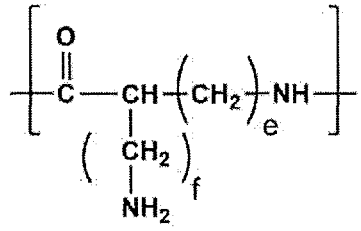
・・・(13)

in the above-mentioned formula (12), c is an integer of 0 to 6 and d is an integer of 0 to 6, and in the above-mentioned formula (13), e is an integer of 0 to 6 and f is an integer of 0 to 6.

In the above-mentioned formula (12), c is an integer of 0 to 6, preferably 0 to 4, most preferably 0 or 4. When c is 0, $(CH_2)_c$ is not included in the above-mentioned formula (12) and CH and NH on both sides of $(CH_2)_c$ are directly single bonded.

In the above-mentioned formula (12), d is an integer of 0 to 6, preferably 0 to 5, most preferably 0 to 4. When d is 0, $(CH_2)_d$ is not included in the above-mentioned formula (12), and CH and NH above and below $(CH_2)_d$ are directly single bonded.

In addition, c and d in the respective ureido group-containing structures represented by the formula (12) in the above-mentioned ureido group-containing repeating sequences E may be the same or different.

Examples of the ureido group-containing repeating sequence E represented by the above-mentioned formula (12) include a ureido group-containing repeating sequence E wherein c is 0 and d is 4 (a-polylysine), a ureido group-containing repeating sequence E wherein c is 0 and d is 3 (polyornithine), a ureido group-containing repeating sequence E wherein c is 4 and d is 0 ($\varepsilon$-polylysine), ureido group-containing repeating sequence E wherein c is 3 and d is 0, and the like. In view of easy availability, a ureido group-containing repeating sequence E wherein c is 0 and d is 4, a ureido group-containing repeating sequence E wherein c is 0 and d is 3, or a ureido group-containing repeating sequence E wherein c is 4 and d is 0 is preferred.

In the hydrophilic repeating sequence F represented by the above-mentioned formula (13), e is an integer of 0 to 6, preferably 0 to 4, most preferably 0 or 4. When e is 0, $(CH_2)_e$ is not included in the above-mentioned formula (13) and CH and NH on both sides of $(CH_2)_e$ are directly single bonded.

In the hydrophilic repeating sequence F represented by the above-mentioned formula (13), f is an integer of 0 to 6, preferably 0 to 5, most preferably 0 to 4. When f is 0, $(CH_2)_f$ is not included in the above-mentioned formula (13) and CH and NH above and below $(CH_2)_f$ are directly single bonded.

In addition, e and f in the respective hydrophilic structures represented by the formula (13) in the above-mentioned hydrophilic repeating sequences F may be the same or different.

Examples of the hydrophilic repeating sequence F represented by the above-mentioned formula (13) include a hydrophilic repeating sequence F wherein e is 0 and f is 4 ($\alpha$-polylysine), a hydrophilic repeating sequence F wherein e is 0 and f is 3 (polyornithine), a hydrophilic repeating sequence F wherein e is 4 and f is 0 ($\varepsilon$-polylysine), hydrophilic repeating sequence F wherein e is 3 and f is 0, and the like. In view of easy availability, a hydrophilic repeating sequence F wherein e is 0 and f is 4, a hydrophilic repeating sequence F wherein e is 0 and f is 3, or a hydrophilic repeating sequence F wherein e is 4 and f is 0 is preferred.

In order to set the upper critical solution temperature of the above-mentioned ureido group-containing polymer with polypeptide as the main chain to 4 to 40°C, ureido group-containing repeating sequences such as the above-mentioned ureido group-containing repeating sequence E and the like are preferably not less than 30 mol%, more preferably not less than 40 mol%, further preferably not less than 50 mol%, of the whole sequences.

When the above-mentioned ureido group-containing polymer has a structure including the above-mentioned ureido group-containing repeating sequence E and the above-mentioned hydrophilic repeating sequence F, a molar ratio (number of moles) of the above-mentioned ureido group-containing repeating sequence E and the above-mentioned hydrophilic repeating sequence F is preferably within the range of $0.3 \leq E/(E+F)$. More preferably the relationship of $0.4 \leq E/(E+F)$ is satisfied, and particularly preferably, the relationship of $0.5 \leq E/(E+F)$ is satisfied.

The molecular weight of the above-mentioned ureido group-containing polymer can be determined as appropriate by adjusting the polymerization conditions and the like so that the required properties can be exhibited, and it is

generally about 1,000 to 5,000,000 in number average molecular weight. In order to increase the cell separation efficiency of the cell detachment agent of the present invention, it is preferably 10,000 to 2,000,000, more preferably 20,000 to 1,000,000. The molecular weight of the above-mentioned ureido group-containing polymer is preferably not more than 5,000,000 because the solubility of the cell detachment agent in water increases to facilitate its use as a cell detachment agent.

<Production method of ureido group-containing polymer - 1>

The method for producing the above-mentioned ureido group-containing polymer is already known and, for example, the method described in JP-B-5800323 can be used. Specifically, a method including dissolving a primary amine-containing polymer in a solvent and reacting same with cyanate (MCNO) can be used.

The above-mentioned primary amine-containing polymer is a polymer having a primary amino group in the side chain thereof.

The main chain of the above-mentioned primary amine-containing polymer is not particularly limited, and may be the same as those listed as examples of the main chain of the aforementioned ureido group-containing polymer.

The molecular weight of the above-mentioned primary amine-containing polymer can be determined as appropriate by adjusting the polymerization conditions and the like so that the required properties can be exhibited, and it is generally about 1,000 to 5,000,000 in number average molecular weight. In order to increase the cell separation efficiency of the cell detachment agent of the present invention, it is preferably 10,000 to 2,000,000, more preferably 20,000 to 1,000,000. The molecular weight of the above-mentioned primary amine-containing polymer is preferably not more than 5,000,000 because the solubility of the cell detachment agent in water increases to facilitate its use as a cell detachment agent.

Examples of the solvent used to dissolve the above-mentioned primary amine-containing polymer include water, buffers such as imidazole-buffer and the like, organic solvents, and mixtures of these. The organic solvent is not particularly limited as long as the primary amine-containing polymer is dissolved. Examples thereof include alcohols such as methanol, ethanol (EtOH), 1-propanol, 2-propanol, 1-butanol, and the like; acetonitrile, formamide, N,N-dimethylformamide (DMF), dimethyl sulfoxide (DMSO), tetrahydrofuran(THF), 1,4-dioxane, and the like. The concentration of the primary amine-containing polymer in the primary amine-containing polymer solution to be used in the reaction is not limited. It is generally 1 to 80 wt%, preferably 2 to 50 wt%, more preferably 5 to 40 wt%. The concentration of the above-mentioned primary amine-containing polymer is preferably not more than 80 wt% because the solution viscosity does not increase too much and the reactivity with the above-mentioned cyanate can be easily maintained.

Preferred examples of the cyanate (MCNO) to be reacted with the above-mentioned primary amine-containing polymer include alkali metal salts of cyanic acid such as potassium cyanate, sodium cyanate, and the like. Potassium cyanate is preferred. While the proportion of cyanate to be used is not particularly limited, it is preferably not less than 0.4 mol per 1 mol of the primary amino group in the above-mentioned primary amine-containing polymer. When the proportion of cyanate to be used is not less than 0.4 mol, ureido groups can be more sufficiently introduced, and the upper limit critical solution temperature can be more easily achieved.

The reaction between the above-mentioned primary amine-containing polymer and cyanate (MCNO) is preferably performed with stirring. While the reaction temperature is not particularly limited, it is desirably maintained at 0 to 100°C, more preferably 30 to 60°C. When the reaction temperature is not less than 0°C, the reaction tends to proceed more sufficiently, and when the reaction temperature is not more than 100°C, the risk of decomposition of the raw materials and further, undesired side reactions becomes less. The reaction time is not particularly limited either, and a solution of the above-mentioned ureido group-containing polymer can be obtained in generally 1 to 48 hr, preferably 1 to 25 hr. When the reaction time is not less than 1 hr, the reaction tends to proceed more sufficiently, and when the reaction time is not more than 48 hr, the risk of decomposition of the raw materials and further, undesired side reactions becomes less.

The obtained above-mentioned ureido group-containing polymer can be used in the next step as it is without purification, or preferably can also be isolated and purified by treatments such as reprecipitation, gel filtration chromatography, and dialysis.

<Production method of the above-mentioned ureido group-containing polymer - 2>

The above-mentioned ureido group-containing polymer can also be produced, for example, by radical polymerization of ethylenically-unsaturated ureido monomers such as the ureido monomer represented by the following formula (14) and the like.

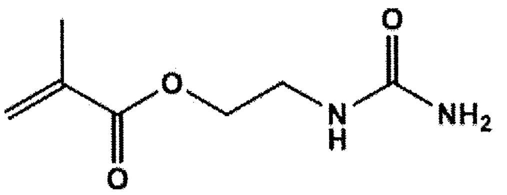

・・・(14)

Radical polymerization of the above-mentioned ethylenically-unsaturated ureido monomers can also be copolymerization with other monomers.

Radical polymerization of the above-mentioned ethylenically-unsaturated ureido monomers (and the above-mentioned other monomers) can also be performed by bulk polymerization, or a solvent can be added to the polymerization. The solvent is not particularly limited as long as the above-mentioned ethylenically-unsaturated ureido monomer (and the above-mentioned other monomers) can be dissolved, and general solvents can be used. For example, the solvent is selected from polar aprotic solvents such as acetone, dioxane, N,N-dimethylformamide, dimethyl sulfoxide, tetrahydrofuran, and the like, polar protic solvents such as methanol, ethanol, water, and the like, and mixtures of these.

Radical polymerization of the above-mentioned ethylenically-unsaturated ureido monomers (and the above-mentioned other monomers) can be performed by thermal polymerization or photopolymerization. The above-mentioned thermal polymerization can be performed, for example, using a thermal polymerization initiator. As the thermal polymerization initiator, for example, a peroxide radical initiator (benzoyl peroxide, ammonium persulfate, etc.) or an azo radical initiator (azobisisobutyro nitrile (AIBN), 2,2'-azobis-dimethylvaleronitrile (ADVN), etc.), 2,2'-azobiscyanovaleric acid (ACVA), azobis [2-(2-imidazolin-2-yl)propane]dihydrochloride (VA-044), water-soluble or oil-soluble redox radical initiator (consisting of dimethylaniline and benzoyl peroxide), or the like can be used. The amount of the radical initiator to be used is preferably generally 0.01 to 10 parts by mass per total 100 parts by mass of the above-mentioned ethylenically-unsaturated ureido monomer (and the above-mentioned other monomers). The polymerization temperature and polymerization time can be appropriately selected and determined according to the kind of the radical initiator, the presence or absence and the kind of other monomers, and the like. For example, when the radical polymerization of the above-mentioned ethylenically-unsaturated ureido monomers (and the above-mentioned other monomers) is performed using AIBN and the like, a polymerization temperature of 40 to 90°C and a polymerization time of about 2 to 48 hr are appropriate.

The above-mentioned photopolymerization can be performed, for example, by irradiation of ultraviolet (UV) at a wavelength of 254 nm or electron beam (EB) at an acceleration voltage of 150 to 300 kV, and the like. In this case, the use of photopolymerization initiator is optional, but is preferred from the aspect of reaction time. As the photopolymerization initiator, 2-hydroxy-2-methyl-1-phenyl-1-propanone, 1-hydroxycyclohexyl phenyl ketone, and the like can be mentioned. From the aspects of solubility and the like, 2-hydroxy-2-methyl-1-phenyl-1-propanone is preferred.

In the radical polymerization of the above-mentioned ethylenically-unsaturated ureido monomers (and the above-mentioned other monomers), a chain transfer agent can also be used. As the above-mentioned chain transfer agent, 2-mercaptoethanol, 1-mercapto-2-propanol, 3-mercapto-1-propanol, p-mercaptophenol, mercaptoacetic acid, 2-mercaptopropionic acid, 3-mercaptopropionic acid, 2-mercaptonicotinic acid, and the like can be mentioned.

The radical polymerization of the above-mentioned ethylenically-unsaturated ureido monomers (and the above-mentioned other monomers) can also be performed by a living radical polymerization method. Specifically, an atom transfer radical polymerization method (ATRP method), a reversible addition-fragmentation chain transfer polymerization method (RAFT polymerization method), a nitroxide-mediated polymerization method (NMP method), or the like can be used. In particular, for use in the raw materials of the cell detachment agent of the present invention, the reversible addition-fragmentation chain transfer polymerization method (RAFT polymerization method) is preferred because no metals are used and the enzyme activity is not reduced.

As the above-mentioned RAFT polymerization method, known methods can be utilized and, for example, the methods described in WO99/31144, WO98/01478, US Patent No. 6,153,705, and the like are effective. When the radical polymerization of the above-mentioned ethylenically-unsaturated ureido monomers (and the above-mentioned other monomers) is performed using the RAFT polymerization, it can be performed by adding a RAFT agent to general radical polymerisation. The above-mentioned RAFT agent is selected from 4-cyanopentanoic acid dithiobenzoate, 2-cyano-2-propylbenzodithioate, benzylbenzodithioate, 2-phenyl-2-propylbenzodithioate, methyl 2-phenyl-2-(phenyl-carbonothioylthio)acetate, 4-cyano-4-(phenylcarbonothioylthio)pentanoic acid N-succinimidyl ester, 4-cyano-4-(dodecylsulfanyl-thiocarbonyl)sulfanylpentanoic acid, 4-cyano-4-(dodecylsulfanyl-thiocarbonyl)sulfanylpentanol, 2-cyano-2-propyldodecyl trithiocarbonate, 2-(dodecylthiocarbonylthioylthio)-2-methylpropionic acid, 4-cyano-4-(dodecylsulfanyl-thiocarbonyl)sulfanylpentanoic acid polyethylene glycol methyl ether ester, 2-(dodecylthiocarbonothioylthio)-2-methylpropionic acid-3-azido-1-propanol ester, benzyl 1H-pyrrole-1-carbodithioate, 2-cyanopropan-2-yl-N-methyl-N-pyridine-4-ylcarbodithioate, propionic acid ethyl-2-ethyl xanthate, and the like. From the aspect of the control of the polymerization of the above-mentioned ureido monomers (and the above-mentioned other monomers), 4-cyanopentanoic acid dithiobenzoate, 4-cyano-4-(phenylcarbonothioylthio)pentanoic acid N-succinimidyl ester, 4-cyano-4-(dodecylsulfanyl-thiocarbonyl)sulfanylpentanoic acid, 4-cyano-4-(dodecylsulfanyl-thiocarbonyl)sulfanylpentanol, or 2-(dodecylthiocarbonothioylthio)-2-methylpropionic acid-3-azido-1-propanol ester is preferred. Furthermore, the above-mentioned ethylenically-unsaturated ureido monomers can form random copolymers or block copolymers with the above-mentioned other monomers, but random copolymer is preferred to bring out the property of the above-mentioned ureido group-containing polymer.

<Bindable ureido group-containing polymer>

The above-mentioned bindable ureido group-containing polymer is a polymer in which the above-mentioned separation carrier-binding site is added to the above-mentioned ureido group-containing polymer.

The above-mentioned separation carrier-binding site is a site that can be bind to the above-mentioned separation carrier and is not particularly limited as long as it is generally used in this field. Examples thereof include hydrophobicity substituent, biotin residue, desthiobiotin residue, avidin residue, streptavidin residue, amino group, carboxyl group, mercapto group, glutathione residue, glutathione transferase residue, lectin residue, polysaccharides residues such as cyclodextrin residue and the like, adamantyl group, phenylboronic acid residue, and the like, preferably biotin residue, streptavidin residue, amino group, carboxyl group, mercapto group, cyclodextrin residue, adamantyl group, phenylboronic acid residue, and the like.

The above-mentioned separation carrier-binding site may be contained at the terminal or in the repeating sequence (side chain and the like) of the above-mentioned ureido group-containing polymer. As a method for containing the above-mentioned separation carrier-binding site in the above-mentioned ureido group-containing polymer, addition, click reaction, amidation, esterification, thioesterification, carbonization, Schiff base formation reaction, reductive amination, radical-mediated coupling reaction, Deals Alder reaction, disulfidation, Michael addition reaction, ene-thiol reaction, aromatic boron compound-mediated coupling reaction, tertiary sulfonium formation reaction, quaternary ammonium formation reaction, and the like can be mentioned. Click reaction, amidation, or esterification is preferred due to their high versatility.

The content of the above-mentioned separation carrier-binding site is not particularly limited as long as the above-mentioned bindable ureido group-containing polymer is bound with the above-mentioned separation carrier. When it is contained in the terminal of the above-mentioned ureido group-containing polymer, 0.2 molecule to 10 molecules of the above-mentioned separation carrier-binding site only needs to be contained in 1 molecule, preferably 0.2 molecule to 4 molecules, further preferably 0.2 molecule to 2 molecules. When not more than 10 molecules are contained, crosslinking between separation carriers is less likely to occur. When the above-mentioned separation carrier-binding site is contained in the repeating sequence of the above-mentioned ureido group-containing polymer, 0.01 to 20 units of the above-mentioned separation carrier-binding site only needs to be contained per 100 repeating units, preferably 0.01 to 10 units, more preferably 0.1 to 5 units. When not more than 20 units are contained, crosslinking between separation carriers is less likely to occur.

<Production method of bindable ureido group-containing polymer containing separation carrier-binding site at terminal>

When a separation carrier-binding site is introduced into the terminal of the above-mentioned ureido group-containing polymer, the ureido group-containing polymer can also be synthesized by a living radical polymerization method. Specifically, the atom transfer radical polymerization method (ATRP method), the reversible addition-fragmentation chain transfer polymerization method (RAFT polymerization method), the nitroxide-mediated polymerization method (NMP method), or the like can be used. In particular, for the use of the cell detachment agent of the present invention, the reversible addition-fragmentation chain transfer polymerization method (RAFT polymerization method) is preferred because no metals are used and the enzyme activity is not reduced.

As the above-mentioned RAFT polymerization method, known methods can be utilized and, for example, the methods described in WO99/31144, WO98/01478, US Patent No. 6,153,705, and the like are effective. When the radical polymerization of the above-mentioned ureido group-containing polymer is performed using the RAFT polymerization, it can be performed by adding a RAFT agent to general radical polymerization. The above-mentioned RAFT agent is selected from 4-cyanopentanoic acid dithiobenzoate, 2-cyano-2-propylbenzodithioate, benzylbenzodithioate, 2-phenyl-2-propylbenzodithioate, methyl 2-phenyl-2-(phenyl-carbonothioylthio)acetate, 4-cyano-4-(phenylcarbonothioylthio)pentanoic acid N-succinimidyl ester, 4-cyano-4-(dodecylsulfanyl-thiocarbonyl)sulfanylpentanoic acid, 4-cyano-4-(dodecylsulfanyl-thiocarbonyl)sulfanylpentanol, 2-cyano-2-propyldodecyl trithiocarbonate, 2-(dodecylthiocarbonylthioylthio)-2-methylpropionic acid, 4-cyano-4-(dodecylsulfanyl-thiocarbonyl)sulfanylpentanoic acid polyethylene glycol methyl ether ester, 2-(dodecylthiocarbonothioylthio)-2-methylpropionic acid-3-azido-1-propanol ester (N3-DTPA), benzyl 1H-pyrrole-1-carbodithioate, 2-cyanopropan-2-yl-N-methyl-N-pyridine-4-ylcarbodithioate, propionic acid ethyl-2-ethyl xanthate, and the like. Due to the easiness of introduction of a separation carrier-binding site into the terminal of the above-mentioned

ureido group-containing polymer, 4-cyanopentanoic acid dithiobenzoate, 4-cyano-4-(phenylcarbonothioylthio)pentanoic acid N-succinimidyl ester, 4-cyano-4-(dodecylsulfanyl-thiocarbonyl)sulfanylpentanoic acid, 4-cyano-4-(dodecylsulfanyl-thiocarbonyl)sulfanylpentanol, or 2-(dodecylthiocarbonothioylthio)-2-methylpropionic acid-3-azido-1-propanol ester is preferred.

when a separation carrier-binding site is introduced into the terminal of the above-mentioned ureido group-containing polymer, the ureido group-containing polymer can also be synthesized by a living anion polymerization method. It is a method of ring-opening polymerization using a polymerization initiator such as α-amino acid-N-carboxyanhydride (NCA) in which the amino group of ornithine or lysine is protected, N-diphenylcarbonato amino acid (DPC), or the like.

As the above-mentioned bindable ureido group-containing polymer, for example, the above-mentioned ureido group-containing polymer represented by the following formula (15) in which an ethylenically-unsaturated polymer is the main chain of the above-mentioned ureido group-containing polymer and a biotin residue at the terminal of the above-mentioned ureido group-containing polymer is a separation carrier-binding site can be mentioned:

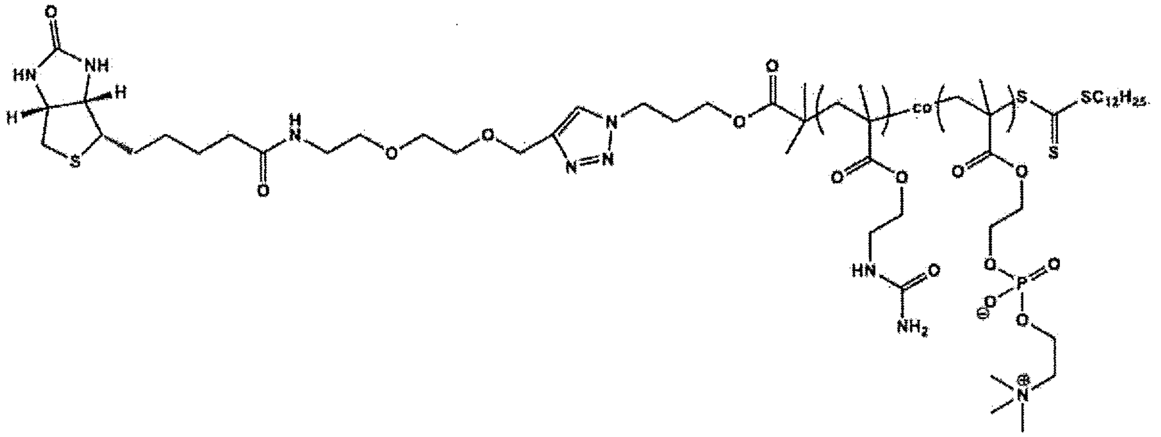

・・・(15)

Examples of the above-mentioned bindable ureido group-containing polymer include the above-mentioned bindable ureido group-containing polymer represented by the following formula (16) in which polyallylamine is the main chain of the above-mentioned ureido group-containing polymer and a biotin residue in the side chain of the above-mentioned ureido group-containing polymer is a separation carrier-binding site.

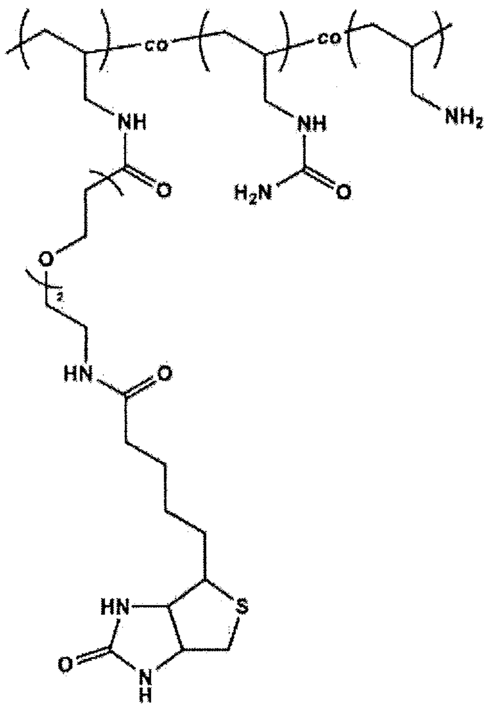

• • • (16)

Examples of the above-mentioned bindable ureido group-containing polymer include the above-mentioned bindable ureido group-containing polymer represented by the following formula (17) in which polyallylamine is the main chain of the above-mentioned ureido group-containing polymer and an amino group in the side chain of the above-mentioned ureido group-containing polymer is a separation carrier-binding site.

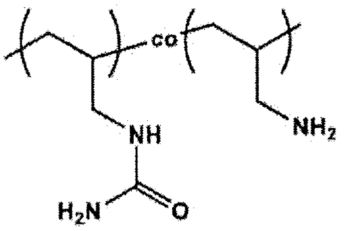

• • • (17)

Examples of the above-mentioned bindable ureido group-containing polymer include the above-mentioned bindable ureido group-containing polymer represented by the following formula (18) in which polypeptide is the main chain of the above-mentioned ureido group-containing polymer and a biotin residue in the side chain of the above-mentioned ureido group-containing polymer is a separation carrier-binding site.

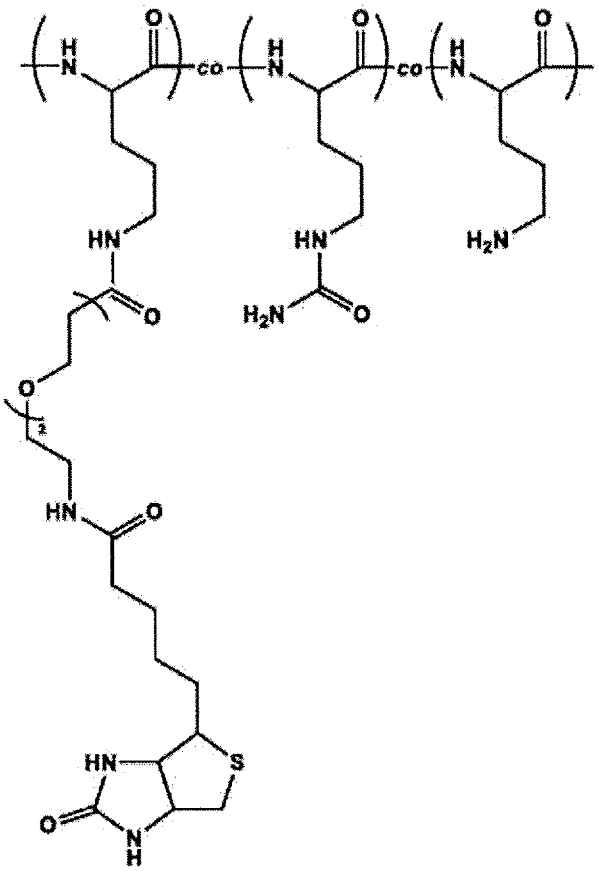

・・・(18)

<Upper critical solution temperature (UCST) of bindable ureido group-containing polymer>

The above-mentioned upper critical solution temperature (UCST) is the boundary temperature between the temperature at which the above-mentioned bindable ureido group-containing polymer is insolubilized in water or a medium to form an insoluble phase, and the temperature at which the above-mentioned bindable ureido group-containing polymer dissolves in water or a medium to form a dissolved phase. The upper critical solution temperature (UCST) is not particularly limited but needs to be a temperature suitable for cell culture, which is 2 to 60°C, preferably 4 to 50°C, more preferably 4 to 40°C.

As mentioned above, the upper critical solution temperature (UCST) of the above-mentioned ureido group-containing polymer is almost the same as the upper critical solution temperature (UCST) of the above-mentioned bindable ureido group-containing polymer.

The above-mentioned upper critical solution temperature (UCST) can be determined by dissolving the above-mentioned (bindable) ureido group-containing polymer in water or a medium at a concentration of at least 1 mM, wherein the permeability of visible light in a clear solution in which the compound is completely dissolved is 100%, measuring the permeability of visible light at 500 nm in a quartz cell while decreasing the temperature, and determining the temperature at which the permeability starts to decrease when the temperature of the solution is decreased.

<Cell detachment agent>

The cell detachment agent in the present invention is a material for detaching a cell to be separated from a separation carrier and contains at least the above-mentioned bindable ureido group-containing polymer. In addition, it generally contains water or medium in addition to the bindable ureido group-containing polymer, and may also contain additives such as chelating agent, protein, and the like for more efficient cell separation.

As the above-mentioned water, purified water, pure water, ion exchange water, and the like are preferred, and may be various buffers containing water. As various buffers, buffers normally used in this field can be used as long as they do not cause loss of physiological activity such as enzymatic activity and antigenicity of proteins. For example, phosphate buffer, tris buffer, Good's buffer, glycine buffer, borate buffer, and the like can be mentioned, and a mixture of these may also be used.

The above-mentioned medium is not particularly limited as long as it is suitable for the cell to be separated, and general cell culture medium can be used. Examples thereof include Dulbecco's modified Eagle MEM medium (DMEM), $\alpha$-MEM medium, Roswell Park Memorial Institute (RPMI) medium, F12 medium, TC199 medium, GMEM medium, and the like. These may be mixed or supplements such as fetal bovine serum (FBS), glutamine, bovine serum albumin, human serum albumin, or even antibiotics may be added as necessary.

Examples of the above-mentioned additive include other reagents generally used in this field to improve cell separation efficiency, for example, chelating agents, saccharides, polysaccharides, proteins, salts, vitamins, surfactants, and the like.

Examples of the above-mentioned chelating agent include ethylenediaminetetraacetic acid (EDTA), 1,2-bis(O-aminophenoxide)ethane-N,N,N',N'-tetraacetic acid (BAPTA), bicine, trans-1,2-diaminocyclohexane-N,N,N',N'-tetraacetic acid (CyDTA), diethylenetriaminepentaacetic acid (DTPA), iminodiacetic acid, triethyl enetetramine hexaacetic acid, and the like.

The above-mentioned saccharides refer to monosaccharides, disaccharides, oligosaccharide, and the like. Examples thereof include allose, glucose (grape sugar), altrose, mannose, galactose, psicose, fructose (fruit sugar), sorbose, ribose, xylose, arabinose, and the like as the monosaccharides, xylulose, trehalose, isotrehalose, maltose (malt sugar), cellobiose, isomaltose, and the like as the disaccharides, fructooligosaccharide, galactooligosaccharide, lactosucrose, and deoxyribose, fucose, rhamnose, glucuronic acid, galacturonic acid, glucosamine, galactosamine xylitol, sorbitol, ascorbic acid (vitamin C), glucuronolactone, gluconolactone, and the like.

Examples of the above-mentioned polysaccharide include chitosan, dextran, cyclodextrin, hyaluronic acid, carboxymethylcellulose, hydroxypropylcellulose, hydroxyethylstarch, starch, pullulan, and the like.

Examples of the above-mentioned protein include bovine fetal serum (FBS), antifreeze peptide, bovine serum albumin, collagen, gelatin, casein, and the like.

Examples of the above-mentioned salt include amino acids such as glycine, alanine, serine, threonine, glutamic acid, aspartic acid, glutamine, asparagine, lysine, histidine, and the like, and amino acid salts, peptides such as glycyl glycine and the like, inorganic salts such as phosphate, borate, sulfate, Tris-salt, and the like, flavins, organic acids such as acetic acid, citric acid, malic acid, maleic acid, gluconic acid, and the like, and organic acid salts, and the like.

Examples of the above-mentioned vitamin include vitamin A, vitamin B1, vitamin B2, vitamin B3, vitamin B5, vitamin B6, vitamin B7, vitamin B9, vitamin B12, vitamin C, vitamin-like substances, and the like.

Examples of the above-mentioned surfactant include Triton, polyoxyethylene alkyl ether, Tween 20, Pluronic, and the like.

The content of the above-mentioned bindable ureido group-containing polymer contained in the cell detachment agent of the present invention is not particularly limited as long as it can be used for separation of cells. It is preferably not less than 0.01 mass %, more preferably not less than 0.05 mass %, of the cell detachment agent.

The above-mentioned additives contained in the cell detachment agent of the present invention are not particularly limited as long as it can be used for separation of cells. The content is preferably not less than 0.01 mass %, more preferably not less than 0.05 mass %, relative to the above-mentioned bindable ureido group-containing polymer.

<Separation carrier>

The material of the above-mentioned separation carrier is not particularly limited and plastic, polysaccharides, metal, metal with magnetism, ceramics, or the like is used. A plurality of these materials may be used. Examples of the plastic include acrylic polymers such as polymethyl methacrylate and the like, various silicone rubbers such as polydimethylsiloxane and the like, polystyrene, poly(ethylene terephthalate), polycarbonate, and the like.

The shape of the separation carrier can be, for example, container, plate, particle, and fiber shapes, as well as porous shape with holes and grooves. Among these, container, plate, particle, fiber, and particle with magnetism (magnetic particle) shapes are preferred for ease of handling during cell separation, and magnetic particles are particularly preferred.

When the above-mentioned separation carrier is a magnetic particle, though free of particular limitation, the average particle size is preferably not less than 0.9 nm and less than 5000 nm, and in order to increase recognition of the object, an average particle size of not less than 2.9 nm and less than 4000 nm is particularly preferred. Examples of the material of the magnetic particles include magnetite, nickel oxide, ferrite, cobalt iron oxide, barium ferrite, carbon steel, tungsten steel, KS steel, rare earth cobalt magnet, hematite, and the like.

The surface of the above-mentioned separation carrier may have various functional groups (e.g., hydroxyl group, tosyl group, carboxyl group, amino group, epoxy group, mercapto group, sulfide group, polyhydric alcohol residue, and the like), and may also have on the surface residues of various compounds (e.g., residues of proteins such as streptavidin, gelatin, collagen, fibronectin, and the like, residues of polysaccharides such as dextran, pullulan, chitosan, cyclodextrin, and the like, residues of chelating agents such as ethylenediamine, and residues of surfactants such as polysorbate).

The combination of the above-mentioned separation carrier surface and the above-mentioned separation carrier-binding site is not particularly limited as long as they can be bound. The combination of a streptavidin residue and a biotin residue, the combination of a streptavidin residue and a desthiobiotin residue, the combination of a tosyl group and an amino group, the combination of an epoxy group and an amino group, the combination of a carboxyl group and an amino group, the combination of a cyclodextrin residue and an adamantyl group, the combination of a polyhydric alcohol residue and a phenylboronic acid residue, and the like can be mentioned. In view of easy availability, the combination of a streptavidin residue and a biotin residue, the combination of a streptavidin residue and a desthiobiotin residue, the combination of a tosyl group and an amino group, the combination of an epoxy group and an amino group, or the combination of a carboxyl group and an amino group is preferred, and the combination of a streptavidin residue and a biotin residue, the combination of a streptavidin residue and a desthiobiotin residue, or the combination of a tosyl group and an amino group is most preferred.

<Cell separation method>

Using the cell detachment agent of the present invention, separation of cells to be separated from a cell population containing the cells to be separated can be performed by a cell separation method including the following [step A], [step B], [step C], and [step D].

[Step A]

A step of adding a substance having a separation carrier-binding site and recognizing a surface antigen of the cell to be separated to a cell population containing the cell to be separated to cause a reaction with the surface antigen of the cell to be separated, thus obtaining a complex of the cells to be separated-substance recognizing the antigen.
As the above-mentioned cell, established cells for culture, fertilized egg and ovum cell of an animal (including human), and the like can be mentioned. In addition, animal (including human) cells such as stem cells (e.g., sperm cell, ES cell, iPS cell, mesenchymal stem cell, hematopoietic stem cell, neural stem cell, cord blood cell, and the like), hepatocyte, nerve cell, cardiomyocyte, vascular endothelial cell, vascular smooth muscle cell, blood cell, and the like or plant cells can also be mentioned.
The above-mentioned cell to be separated refers to a population of cells obtained as a result of cell separation using the cell detachment agent of the present invention, and may be a single type or a cell population consisting of multiple types of cells.
The above-mentioned cell population containing the cells to be separated refers to a cell population preferably containing not less than 0.1% of the cells to be separated, and the cell types included therein are not restricted.
The above-mentioned surface antigen may be present in any of blood, body fluid, tissue section, cell aggregate, suspended cells, adherent cells, and the like, and they may be viable or dead cells.
Examples of the above-mentioned substance recognizing the surface antigen include any antibody, antibody derivative, fragmented antibody, or fragmented antibody derivative that is against an antigen expressed intracellularly or extracellularly, TCR molecules targeting peptide/MHC complexes, cell adhesion receptor molecules, receptors for co-stimulatory molecules, artificially engineered binding molecules, protein A, lectins, peptides targeting cell surface molecules, aptamers, and the like.
As the substance having the above-mentioned separation carrier-binding site and recognizing the antigen of the cells to be separated, commercially available reagents can also be used. It can also be prepared separately according to the substance recognizing the antigen to be used. As methods for including the above-mentioned separation carrier-binding site in the above-mentioned substance recognizing the surface antigen, addition, click reaction, amidation, esterification, thioesterification, carbonization, Schiff base formation reaction, reductive amination, radical-mediated coupling reaction, Diels-Alder reaction, disulfidation, Michael addition reaction, ene-thiol reaction, aromatic boron compound-mediated coupling reaction, tertiary sulfonium formation reaction, quaternary ammonium formation reaction, and the like can be mentioned. Click reaction, amidation, or esterification is preferred due to their high versatility.
The reaction between the above-mentioned substance having the separation carrier-binding site and recognizing the surface antigen of the cells to be separated and the above-mentioned surface antigen of the cells to be separated is not particularly limited as long as it does not adversely affect the cells to be separated. The reaction temperature is generally 2°C to 25°C, preferably 2°C to 20°C, more preferably 2°C to 10°C. Also, the reaction time is not particularly limited and it is generally 10 min to 2 hr, preferably 10 min to 1 hr, more preferably 10 min to 30 min.
The reaction between the above-mentioned substance having the separation carrier-binding site and recognizing the surface antigen of the cells to be separated and the above-mentioned surface antigen of the cells to be separated can be performed in, for example, a cell separation buffer. It is not particularly limited as long as it does not adversely affect the cells to be separated and buffers and media generally used in this field can be used. Examples thereof include phosphate buffer, tris buffer, Good's buffer, glycine buffer, borate buffer, and the like, and a mixture of

these may also be used. Phosphate buffer is preferred. Also, Dulbecco's modified Eagle MEM medium (DMEM), α-MEM medium, Roswell Park Memorial Institute (RPMI) medium, F12 medium, TC199 medium, GMEM medium, and the like can be mentioned. These may be mixed or supplements such as fetal bovine serum (FBS), glutamine, or antibiotics may also be added as necessary, but serum-free medium is preferred to avoid impairing the reactivity of the substance recognizing the antigen. In addition, various chelating agents can also be used to prevent cell aggregation. As the chelating agent, the same chelating agents as those recited as examples of the chelating agent as an additive in the above-mentioned cell detachment agent can be mentioned, with preference given to ethylenediaminetetraacetic acid (EDTA). The concentration of chelating agent in the cell separation buffer is 0.1 mM to 20 mM, preferably 0.5 mM to 10 mM, more preferably 1 mM to 5 mM. When the concentration of the chelating agent is not less than 0.1 mM, a cell aggregation suppressive effect is more easily expressed. On the other hand, when it is not more than 20 mM, cytotoxicity and the like are less likely to be exhibited.

Furthermore, in order to promote the reaction between the above-mentioned substance having the separation carrier-binding site and recognizing the surface antigen of the cells to be separated and the above-mentioned surface antigen of the cells to be separated, polysaccharides, proteins, surfactants, and the like may also be added. As the above-mentioned polysaccharides, the same polysaccharides as those recited as examples of the polysaccharide as an additive in the above-mentioned cell detachment agent can be mentioned, and as the above-mentioned protein, the same proteins as those recited as examples of the protein as an additive in the above-mentioned cell detachment agent can be mentioned. As the above-mentioned surfactant, the same surfactants as those recited as examples of the surfactant as an additive in the above-mentioned cell detachment agent can be mentioned, and dextran, bovine serum albumin, or Tween20 is preferred, and bovine serum albumin is more preferred. Polysaccharides, proteins, surfactants, and the like in a cell separation buffer are not particularly limited as long as they can be used for cell separation, but not less than 0.01 mass% is preferred, and not less than 0.05 mass% is more preferred. When polysaccharides, proteins, surfactants, and the like in a cell separation buffer are within the above-mentioned range, the above-mentioned additives are more likely to exert sufficient effects.

A complex of the cells to be separated-substance recognizing the antigen can be obtained by performing the above [step A]. A specific example of [step A] is a step of reacting a surface antigen of the cells to be separated with a biotinylated antibody.

[Step B]

A step of interacting the complex of the cells to be separated-substance recognizing the antigen obtained in [step A] with a separation carrier via a separation carrier-binding site to obtain a complex of the cells to be separated-separation carrier.

The reaction between the above-mentioned complex of the cells to be separated-substance recognizing the antigen with a separation carrier is not particularly limited as long as it does not adversely affect the cells to be separated. For example, the reaction can be performed using the above-mentioned cell separation buffer. The reaction temperature is generally 2°C to 25°C, preferably 2°C to 20°C, more preferably 2°C to 10°C. By performing the reaction at not more than 25°C, it is easier to prevent cells from taking up surface antigens and antibodies to result in a decrease in the number of cells bound to the separation carrier. Also, the reaction time is not particularly limited and it is generally 10 min to 2 hr, preferably 10 min to 1 hr, more preferably 10 min to 30 min.

A complex of the cells to be separated-separation carrier can be obtained by performing the above [step B]. A specific example of [step B] is a step of binding the cells to be separated to a separation carrier via a biotinylated antibody.

[Step C]

A step of separating only the complex of the cell to be separated-separation carrier obtained in [step B] from the cell population containing the cell to be separated.

A method of separating only the above-mentioned complex of the cells to be separated-separation carrier from the cell population containing the cells to be separated varies depending on the separation carrier. For example, when the separation carrier is a magnetic particle, a method of collecting by magnetic force is generally used. When the separation carrier is a particle such as a latex particle, it can be precipitated by time change or by centrifugation. Furthermore, when the separation carrier is a flat plate, it can simply be removed from the above-mentioned cell separation buffer.

After the above-mentioned separation, washing is preferred, and washing is not limited as long as it does not adversely affect the cells to be separated. For example, the above-mentioned cell separation buffer can also be used.

A complex of the cells to be separated-separation carrier can be separated by performing the above [step C]. A specific example of [step C] is a step of taking out the complex of the cells to be separated-separation carrier from the cell population containing the cells to be separated by magnetism, centrifugation, or the like.

[Step D]

A step of adding the above-mentioned cell detachment agent to the complex of the cell to be separated-separation carrier obtained in [step C] and separating the cell to be separated by temperature change.

The above-mentioned cell detachment agent may be directly added to the above-mentioned complex of the cells to be separated-separation carrier, but it may be mixed with the above-mentioned cell separation buffer and added. The temperature at the time of addition is not limited as long as it is below the upper critical solution temperature (UCST) of the bindable ureido group-containing polymer which is a constituent component of the cell detachment agent to be used. The temperature is preferably 4 to 10°C as the temperature that does not adversely affect the cells. In addition, the reaction time is not particularly limited and is generally 10 min to 2 hr, preferably 10 min to 1 hr, more preferably 10 min to 30 min.

The temperature change in the above-mentioned [step D] refers to heating from a temperature not more than the upper critical solution temperature (UCST) of a bindable ureido group-containing polymer which is a constituent component of the cell detachment agent to a temperature not less than the upper critical solution temperature (UCST). The temperature after heating is more preferably 25 to 40°C as the temperature that does not adversely affect the cells.

The cells to be separated can be separated from the complex of the cells to be separated-separation carrier by performing the above [step D]. A specific example of [step D] is a step of reacting a complex of the cells to be separated-separation carrier with a cell detachment agent, changing the temperature to stop the interaction between the separation carrier and the cells to be separated, and taking out the cells to be separated.

[Example]

The present invention is explained in detail in the following by referring to Examples. The present invention is not limited to the following Examples and the like.

(Synthetic Example 1: Synthesis of ureido monomer (1))

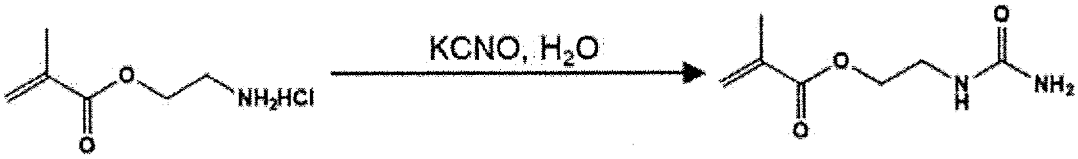


ureido monomer (1)

Aminoethylmethacrylate hydrochloride (20.0 g, 120 mmol) was added into an eggplant flask and dissolved in ion exchange water (90 mL). Potassium cyanate (10.7 g, 132 mmol) was added and the mixture was stirred at room temperature. After stirring for 2 hr, the reaction solution was freeze-dried and resuspended in acetone (200 mL). Thereafter, the insoluble material was filtered off, and the solvent was evaporated from the obtained mixed solution under reduced pressure. Purification thereafter by silica gel chromatography with ethyl acetate/methanol 9/1(v/v) gave ureido monomer (1).

yield: 12 g

$^1$H-NMR(DMSO-d6, 400 MHz)
6.2 ppm($CON\underline{H_2}$), 5.7 ppm($C\underline{H_2}$=C), 5.5 ppm ($C\underline{H_2}$=C, N$\underline{H}$CO), 4.0 ppm($OC\underline{H_2}$), 3.2 ppm($C\underline{H_2}NH$), 2.0 ppm($C\underline{H_3}$)

(Synthetic Example 2-1: Synthesis of ureido group-containing polymer A)

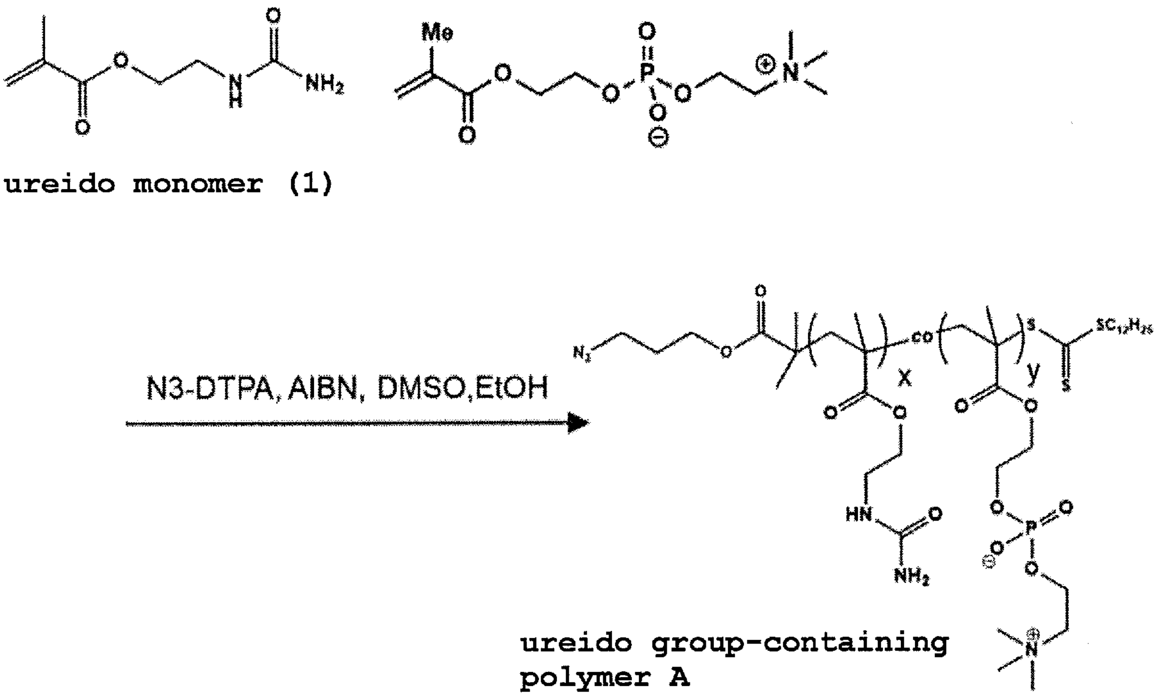


ureido monomer (1)

ureido group-containing polymer A

wherein co indicates that the structure is a copolymer structure of two monomer units in the chemical formula.

Ureido monomer (1) (380 mg), 2-methacryloyloxyethylphosphoryl choline (2) (MPC, 32.8 mg), 2-(dodecylthiocarbonothioylthio)-2-methylpropionic acid-3-azido-1-propanol ester (0.90 mg), and azobisisobutyro nitrile (0.164 mg) were added into a test tube and dissolved in dimethyl sulfoxide (2.4 mL), ethanol (0.6 mL). After bubbling with a nitrogen gas, the reaction mixture was heated to 65°C and reacted for 20 hr. The polymerized solution was reprecipitated with acetone to give ureido group-containing polymer A.

yield: 350 mg

composition ratio (molar ratio) x:y=95:5 (calculated from $^1$H NMR)

$^1$H-NMR($CD_3OD$+$D_2O$, 400 MHz)

4.1 ppm(Ureido:COO-$CH_2$), 3.8-4, 6 ppm(MPC:COO-$CH_2$,$CH_2$-$CH_2$,PO-$CH_2$), 3.6 ppm(MPC:$CH_2$-N), 3.4 ppm (Ureido: $CH_2$-$CH_2$), 3.2 ppm(N($CH_3$)$_3$),1.0-2.4 ppm(polymer main chain)

The number average molecular weight of the obtained ureido group-containing polymer A was determined by gel permeation chromatography using a multi-angle light scattering detector (MALS) manufactured by WYATT as a detector. The pump used was LC-720AD manufactured by Shimadzu Corporation, the detector (differential refractometer) used was RID10A manufactured by Shimadzu Corporation, and the detector (UV) used was SPD-20A. Two columns of TSKGel G3000PWXL and TSKGel G5000PWXL (column size 4.6 mm×25cm) manufactured by Tosoh Corporation were connected and used. As the eluent, distilled water/acetonitrile (5/5 v/v) in which 100 mM sodium nitrate was dissolved was used. The measurement conditions were flow rate 0.6 ml/min, column temperature 40°C, sample concentration 2 mg/mL, injection volume 100 μL. As a result of gel permeation chromatography measurement, the number average molecular weight of the ureido group-containing polymer A was 308,000.

The phase transfer temperature of the obtained ureido group-containing polymer A was measured by an ultraviolet visible spectrophotometer. The ureido group-containing polymer A was dissolved in a cell separation buffer (phosphate buffered saline (PBS)+0.1% bovine serum albumin (BSA)+2 mM EDTA) at a concentration of 2.5 mg/mL. The solution was placed in a quartz cell, the solution temperature was changed within the range of 70°C to 5°C, and the permeability (%) of the solution was measured by the ultraviolet visible spectrophotometer. The permeability (%) of the solution was calculated from the following formula. As a result, the phase transfer temperature of the ureido group-containing polymer A was 20°C.

$$\text{permeability (\%)} = 10^{(-\text{absorbance})}$$

(Synthetic Example 2-2: Synthesis of bindable ureido group-containing polymer A')

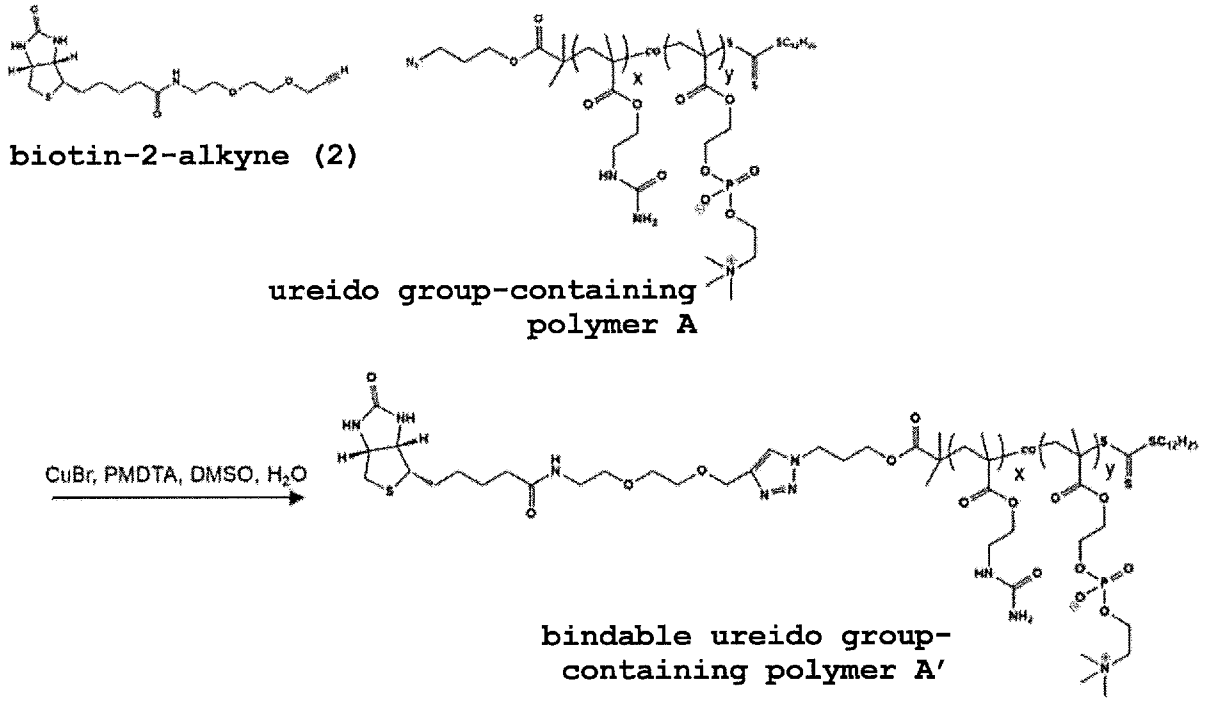


biotin-2-alkyne (2)

ureido group-containing polymer A

bindable ureido group-containing polymer A'

wherein co indicates that the structure is a copolymer structure of two monomer units in the chemical formula.

Ureido group-containing polymer A (300 mg), biotin-2-alkyne (2) (34.1 mg), and copper(I) bromide (13.2 mg) were added into a test tube and dissolved in dimethyl sulfoxide (4 mL), ion exchange water (1 mL). A solution (1 mL) of pentamethyldiethylenetriamine (PMDTA) (16.0 mg) in dimethyl sulfoxide was added to start the reaction. After reaction at room temperature overnight, the mixture was purified by dialysis to give bindable ureido group-containing polymer A'. yield: 300 mg

(Preparation Example 2-3: Preparation of cell detachment agent A")

Bindable ureido group-containing polymer A' was dissolved in a cell separation buffer (phosphate buffered saline (PBS)+0.1% bovine serum albumin (BSA)+2 mM EDTA) at a concentration of 0.8 mg/mL to prepare cell detachment agent A".

(Synthetic Example 3-1: Synthesis of ureido group-containing polymer B)

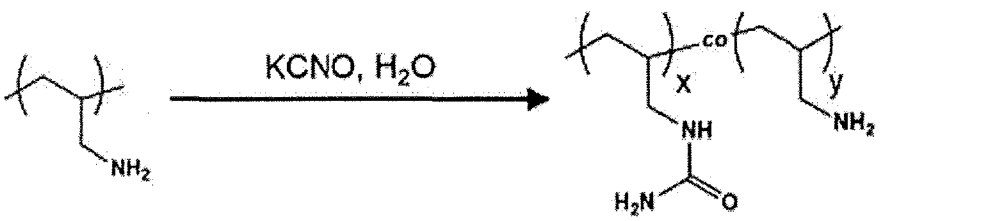


ureido group-containing polymer B

wherein co indicates that the structure is a copolymer structure of two monomer units in the chemical formula.

Polyallylamine (500 mg) with a number average molecular weight of about 30,000 and purchased from Merck

was added into a four-lidded flask and dissolved in ion exchange water (10 mL). The mixture was heated to 50°C, potassium cyanate (611 mg, 7.54 mmol) (0.86 mol per 1 mol of amino group in polyallylamine) was added thereto, and the mixture was reacted for 24 hr. After completion of the reaction, the mixture was purified by dialysis at the same temperature to give ureido group-containing polymer B.

yield: 579 mg
composition ratio (molar ratio) x:y=85:15 (calculated from $^1$H NMR)

The number average molecular weight of the obtained ureido group-containing polymer B was determined in the same manner as in Synthetic Example 2-1. As a result of the measurement by gel permeation chromatography, the number average molecular weight of the ureido group-containing polymer B was 36,000.

The phase transfer temperature of the obtained ureido group-containing polymer B was determined in the same manner as in Synthetic Example 2-1. As a result of the measurement with the ultraviolet visible spectrophotometer, the phase transfer temperature was 30°C.

(Synthetic Example 3-2: Synthesis of bindable ureido group-containing polymer B')

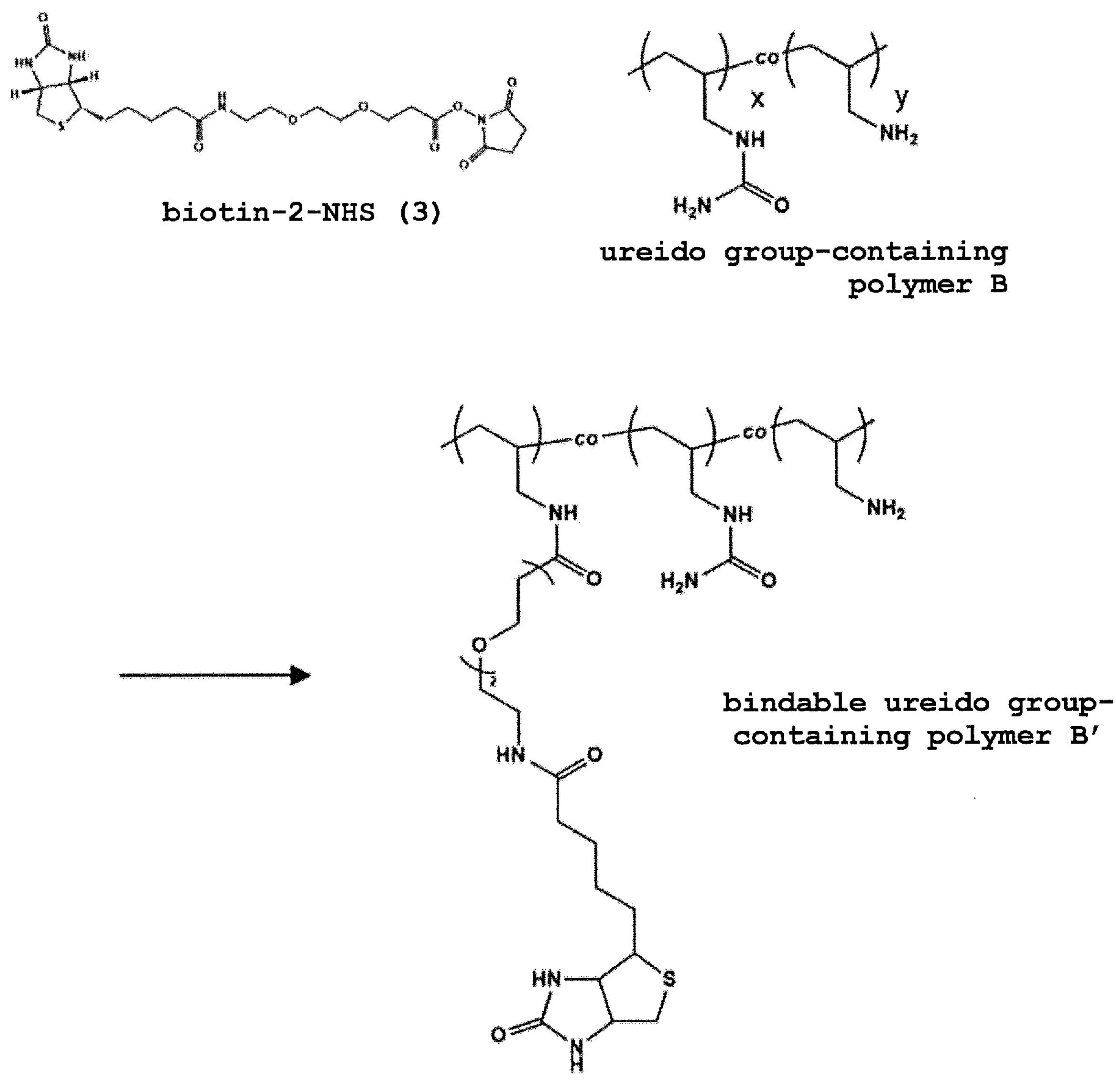

biotin-2-NHS (3)

ureido group-containing polymer B

bindable ureido group-containing polymer B'

wherein co indicates that the structure is a copolymer structure of two or three monomer units in the chemical formula.

Ureido group-containing polymer B (300 mg) and biotin-2-NHS (3) (50 mg, 0.10 mmol) (0.1 mol per 1 mol of amino group in ureido group-containing polymer B) were added into a test tube and dissolved in dimethyl sulfoxide (1 mL). After reaction at room temperature overnight, the mixture was purified by dialysis to give bindable ureido group-containing polymer B'. yield: 326 mg

(Preparation Example 3-3: Preparation of cell detachment agent B")

Bindable ureido group-containing polymer B' was dissolved in a cell separation buffer (phosphate buffered saline (PBS)+0.1% bovine serum albumin (BSA)+2 mM EDTA) at a concentration of 0.8 mg/mL to prepare cell detachment agent B".

(Synthetic Example 4-1: Synthesis of ureido group-containing polymer C)

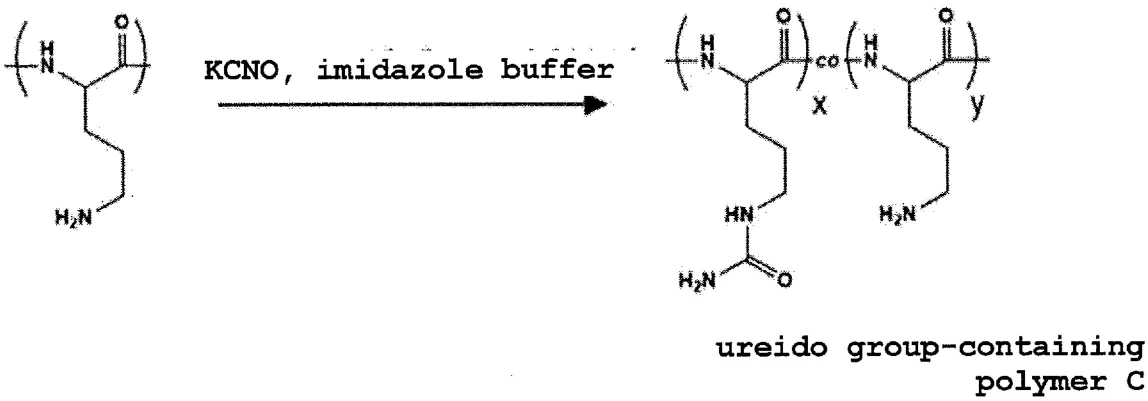


wherein co indicates that the structure is a copolymer structure of two monomer units in the chemical formula.

Poly-L-ornithine (500 mg) with a number average molecular weight of about 150,000 to 300,000 was placed in a test tube and dissolved in 1M imidazole buffer (pH 7) (1 ml). The mixture was heated to 50°C, potassium cyanate (263 mg, 3.25 mmol) (0.86 mol per 1 mol of ornithine residue) was added thereto, and the mixture was reacted for 24 hr. After completion of the reaction, the mixture was purified by dialysis at the same temperature to give ureido group-containing polymer C.

yield: 540 mg
composition ratio (molar ratio) x:y=84:16 (calculated from $^1$H NMR)

The number average molecular weight of the obtained ureido group-containing polymer C was determined in the same manner as in Synthetic Example 2-1. As a result of the measurement by gel permeation chromatography, the number average molecular weight of the ureido group-containing polymer C was 260,000.

The phase transfer temperature of the obtained ureido group-containing polymer C was determined in the same manner as in Synthetic Example 2-1. As a result of the measurement with the ultraviolet visible spectrophotometer, the phase transfer temperature was 20°C.

(Synthetic Example 4-2: Synthesis of bindable ureido group-containing polymer C')

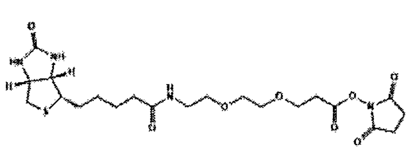

biotin-2-NHS (3)

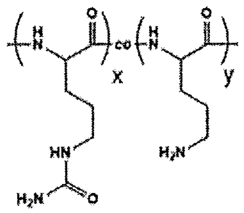

ureido group-containing polymer C

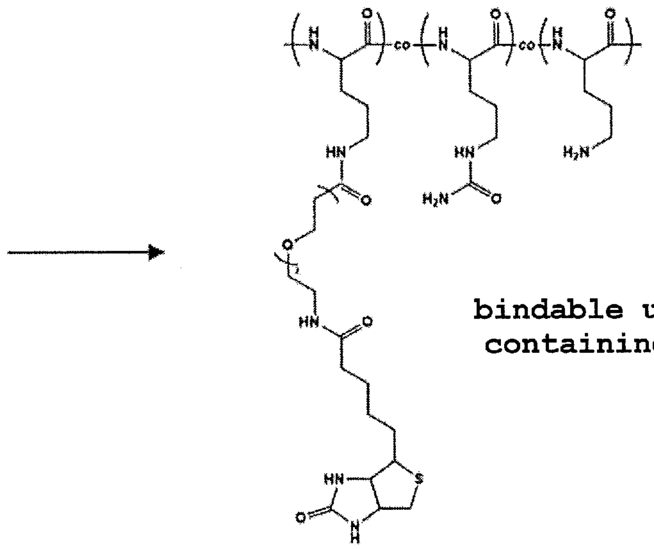

bindable ureido group-containing polymer C'

wherein co indicates that the structure is a copolymer structure of two or three monomer units in the chemical formula.

Ureido group-containing polymer C (300 mg) and biotin-2-NHS (3) (30 mg, 0.06 mmol) (0.1 mol per 1 mol of amino group in ureido group-containing polymer C) were added into a test tube and dissolved in dimethyl sulfoxide (1 mL). After reaction at room temperature overnight, the mixture was purified by dialysis to give bindable ureido group-containing polymer C'. yield: 298 mg

(Preparation Example 4-3: Preparation of cell detachment agent C" )

Bindable ureido group-containing polymer C' was dissolved in a cell separation buffer (phosphate buffered saline (PBS)+0.1% bovine serum albumin (BSA)+2 mM EDTA) at a concentration of 0.8 mg/mL to prepare cell detachment agent C".

(Preparation Example 5: Preparation of cell detachment agent D" )

Ureido group-containing polymer B was dissolved in a cell separation buffer (phosphate buffered saline (PBS)+0.1% bovine serum albumin (BSA)+2 mM EDTA) at a concentration of 0.8 mg/mL to prepare cell detachment agent D".

<Example 1-1>

(Cell separation test using single cell type)

Human-derived leukemia cell line HL-60 ($5\times10^6$ cells/mL, 200 $\mu$L) suspended in serum-free RPMI1640 medium was dispensed into an Eppendorf tube to prepare a cell suspension. The number of cells in the prepared cell suspension was counted (Count A). Biotinylated anti-CD45 antibody (4 $\mu$L) was added to the cell suspension, and the mixture was shaken at 4°C for 10 min. Thereafter, 300 $\mu$L of a cell separation buffer (phosphate buffered saline (PBS)+0.1% bovine serum albumin (BSA)+2 mM EDTA) was added and the mixture was centrifuged at 350 g for 10 min. After centrifugation, the supernatant was removed and the mixture was resuspended in a cell separation buffer (200 $\mu$L). Dynabeads (registered trademark) M-280 Streptavidin ($6\times10^8$ beads/mL, 16 $\mu$L) manufactured by INVITROGEN was added as a separation carrier and the mixture was stirred, followed by shaking at 4°C for 15 min. The beads were collected by magnetism at 4°C, the supernatant was removed, and the beads were washed with a cell separation buffer. A cell detachment agent A" was added and the mixture was reacted at 4°C for 15 min. Thereafter, the mixture was reacted at 37°C for 15 min, collected again by magnetism, the supernatant was recovered (measurement sample X), and the number of cells was counted (count B). The number of cells was counted using a cell counter (Countess 2) manufactured by ThermoFisher, after treatment with trypan blue. The survival rate of measurement sample X was determined using a cell counter (Countess 2) manufactured by ThermoFisher, after separation and culturing at 37°C for 6 hr without washing after separation and after treatment with trypan blue.

The recovery rate of HL-60 cells after cell separation was calculated from the following formula (5). The survival rate was calculated from the following formula (6). The evaluation results are shown in Table 1.

[number 2]

recovery rate = (count B/count A) x 100 (5)

survival rate = (number of HL-60 viable cells in measurement sample X/number of total HL-60 cells in measurement sample X) x 100 (6)

<Examples 1-2 to 1-4>

In the same manner as in Example 1-1 except that the separation carriers and cell detachment agents shown in Table 1 were used, cell separation tests using single cell type were performed. The evaluation results are shown in Table 1.

<Example 2-1>

(Cell separation test using plural cell types)

A plural cell mixture (total number of cells $5\times10^6$ cells/mL, 200 μL) was prepared by mixing HeLa cells and HL-60 cells that were pre-stained with Hoechest 33342, at a ratio of 33:67 in cell number in an Eppendorf tube (measurement sample A). Biotinylated anti-CD45 antibody (4 μL) was added thereto, and the mixture was shaken at 4°C for 10 min. Thereafter, 300 μL of a cell separation buffer (phosphate buffered saline (PBS)+0.1% bovine serum albumin (BSA)+2 mM EDTA) was added and the mixture was centrifuged at 350 g for 10 min. After centrifugation, the supernatant was removed and the mixture was resuspended in a cell separation buffer (200 μL). Dynabeads (registered trademark) M-280 Streptavidin ($6\times10^8$ beads/mL, 16 μL) manufactured by INVITROGEN was added as a separation carrier and the mixture was stirred, followed by shaking at 4°C for 15 min. The beads were collected by magnetism at 4°C, the supernatant was removed at 4°C, and the beads were washed with a cell separation buffer. A cell detachment agent A" was added and the mixture was reacted at 4°C for 15 min. Thereafter, the mixture was reacted at 37°C for 15 min, collected again by magnetism, the supernatant was recovered (measurement sample B). For measurement samples A and B, CD45-positive cells were stained using a fluorescein isothiocyanate (FITC)-labeled secondary antibody. Thereafter, the sample was placed in a Truecount Tube manufactured by BD and analyzed using an LSRFortessa X-20 flow cytometer. The survival rate of measurement sample B was determined using a cell counter (Countess 2) manufactured by ThermoFisher, after separation and culturing at 37°C for 6 hr without washing after separation and after treatment with trypan blue.

The recovery rate of HL-60 cells after cell separation was calculated from the following formula (7). Then the purity was calculated from the following formula (8). The survival rate was calculated from the following formula (9). The evaluation results are shown in Table 2.

recovery rate = (number of total HL-60 cells in measurement sample B/number of total HL-60 cells in measurement sample A) × 100 (7)

purity rate = 100 - (number of HeLa cells in measurement sample B/number of total cells in measurement sample B) $\times$ 100 (8)

survival rate = (number of HL-60 viable cells in measurement sample B/number of total HL-60 cells in measurement sample B) $\times$ 100 (9)

<Examples 2-2 to 2-4>

In the same manner as in Example 2-1 except that the separation carriers and cell detachment agents shown in Table 2 were used, cell separation tests using plural cell types were performed. The evaluation results are shown in Table 2.

<Comparative Example 1>

Human-derived leukemia cell line HL-60 ($5\times10^6$ cells/mL, 200 $\mu$L) suspended in serum-free RPMI1640 medium was dispensed into an Eppendorf tube to prepare a cell suspension. The number of cells in the prepared cell suspension was counted (Count A). Desthiobiotinylated anti-CD45 antibody (4 $\mu$L) was added to the cell suspension, and the mixture was shaken at 4°C for 10 min. Thereafter, 300 $\mu$L of a cell separation buffer (phosphate buffered saline (PBS)+0.1% bovine serum albumin (BSA)+2 mM EDTA) was added and the mixture was centrifuged at 350 g for 10 min. After centrifugation, the supernatant was removed and the mixture was resuspended in a cell separation buffer (200 $\mu$L). Dynabeads (registered trademark) M-280 Streptavidin ($6\times10^8$ beads/mL, 16 $\mu$L) manufactured by INVITROGEN was added as a separation carrier and the mixture was stirred, followed by shaking at 4°C for 15 min. The beads were collected by magnetism at 4°C, the supernatant was removed, and the beads were washed with a cell separation buffer. A biotin-containing cell separation buffer was added and the mixture was reacted at 4°C for 15 min. The mixture was collected again by magnetism, the supernatant was recovered (measurement sample X), and the number of cells was counted (count B). The number of cells was counted using a cell counter (Countess 2) manufactured by ThermoFisher, after treatment with trypan blue. The survival rate of measurement sample X was determined using a cell counter (Countess 2) manufactured by ThermoFisher, after separation and culturing at 37°C for 6 hr without washing after separation and after treatment with trypan blue.

The recovery rate of HL-60 cells after cell separation was calculated from the above-mentioned formula (5). The survival rate was calculated from the above-mentioned formula (6). The evaluation results are shown in Table 1.

<Comparative Example 2>

A plural cell mixture (total number of cells $5\times10^6$ cells/mL, 200 $\mu$L) was prepared by mixing HeLa cells and HL-60 cells that were pre-stained with Hoechest 33342, at a ratio of 33:67 in cell number in an Eppendorf tube (measurement sample A). Desthiobiotinylated anti-CD45 antibody (4 $\mu$L) was added thereto, and the mixture was shaken at 4°C for 10 min. Thereafter, 300 $\mu$L of a cell separation buffer (phosphate buffered saline (PBS)+0.1% bovine serum albumin (BSA)+2 mM EDTA) was added and the mixture was centrifuged at 350 g for 10 min. After centrifugation, the supernatant was removed and the mixture was resuspended in a cell separation buffer (200 $\mu$L). Dynabeads (registered trademark) M-280 Streptavidin ($6\times10^8$ beads/mL, 16 $\mu$L) manufactured by INVITROGEN was added as a separation carrier and the mixture was stirred, followed by shaking at 4°C for 15 min. The beads were collected by magnetism at 4°C, the supernatant was removed at 4°C, and the beads were washed with a cell separation buffer. A biotin-containing cell separation buffer was added and the mixture was reacted at 4°C for 15 min. The mixture was collected again by magnetism, and the supernatant was recovered (measurement sample B). For measurement samples A and B, CD45-positive cells were stained using a fluorescein isothiocyanate (FITC)-labeled secondary antibody. Thereafter, the sample was placed in a Truecount Tube manufactured by BD and analyzed using an LSRFortessa X-20 flow cytometer. The survival rate of measurement sample B was determined using a cell counter (Countess 2) manufactured by ThermoFisher, after separation and culturing at 37°C for 6 hr without washing after separation and after treatment with trypan blue.

The recovery rate of HL-60 cells after cell separation was calculated from the above-mentioned formula (7). Then the purity was calculated from the above-mentioned formula (8). The survival rate was calculated from the above-mentioned formula (9). The evaluation results are shown in Table 2.

As shown in Tables 1 and 2, when the polymers of Examples were used, the recovery rate was improved compared to when desthiobiotinylated antibody was prepared separately and cells were separated using the biotin-containing cell separation buffer. It can also be appreciated that the survival rate of cells separated using the cell detachment agent of the present invention is very high.

From the above, it is appreciated that the expansion and/or contraction of polymer chains due to temperature response can be used as a driving force to detach cells to be separated from a separation carrier, and that a polymer that does not affect the cells after separation, for example, a cell detachment agent containing a bindable ureido group-containing polymer having a separation carrier-binding site capable of binding to a separation carrier in a cell separation method using a surface antigen of cells to be separated is useful. Furthermore, it is also clear that highly versatile biotinylated antibodies can be used in a cell separation method using the cell detachment agent.

[Table 1]

| | separation carrier | cell detachment agent | antibody used for separation | recovery rate (%) | survival rate (%) |
|---|---|---|---|---|---|
| Example 1-1 | Dynabeads (registered trademark) M-280 Streptavidin | cell detachment agent A" | biotinylated anti-CD45 antibody | 21 | 99 |
| Example 1-2 | | cell detachment agent B" | | 20 | 97 |
| Example 1-3 | | cell detachment agent C" | | 19 | 95 |
| Example 1-4 | Dynabeads (registered trademark) M-450 Tosylactivated | cell detachment agent D" | | 22 | 98 |
| Comparative Example 1 | Dynabeads (registered trademark) M-280 Streptavidin | biotin-containing cell separation buffer | desthiobiotinylated anti-CD45 antibody | 12 | 65 |

[Table 2]

| | separation carrier | celldetachment agent | antibody used for separation | recovery rate (%) | purity (%) | survival rate (%) |
|---|---|---|---|---|---|---|
| Example 2-1 | Dynabeads (registered trademark) M-280 Streptavidin | celldetachment agent A" | biotinylated anti-CD45 antibody | 17 | 99 | 99 |
| Example 2-2 | | celldetachment agent B" | | 19 | 99 | 97 |
| Example 2-3 | | celldetachment agent C" | | 20 | 99 | 98 |
| Example 2-4 | Dynabeads (registered trademark) M-450 Tosylactivated | celldetachment agent D" | | 19 | 99 | 95 |
| Comparative Example 2 | Dynabeads (registered trademark) M-280 Streptavidin | biotin-containing cell separation buffer | desthiobiotinylated anti-CD45 antibody | 13 | 99 | 56 |

[Industrial Applicability]

The present invention provides a cell detachment agent that can be applied to broad types of cells in a cell separation method using surface antigen of cells to be separated, and that does not require washing after separation of the cells to be separated, and a cell separation method that uses the cell detachment agent.

This application is based on a patent application No. 2021-109278 filed in Japan (filing date: June 30, 2021), the contents of which are incorporated in full herein.

## Claims

1. A cell detachment agent for detaching a cell to be separated from a separation carrier bound to the cell to be separated, wherein

   the cell detachment agent comprises a bindable ureido group-containing polymer having a side chain structure represented by the following formula (1) and having a separation carrier-binding site capable of binding to the separation carrier at a side chain or terminal thereof, and
   the bindable ureido group-containing polymer has an upper critical solution temperature (UCST) of 4 to 40°C:

   $$-NH-(C=O)-NH_2 \cdots \quad (1).$$

2. The cell detachment agent according to claim 1, wherein the separation carrier-binding site is a biotin residue, a streptavidin residue, an amino group, a carboxyl group, a mercapto group, a cyclodextrin residue, an adamantyl group, or a phenylboronic acid residue.

3. The cell detachment agent according to claim 1 or 2, wherein the ureido group-containing polymer has a main chain which is an ethylenically-unsaturated polymer, a polyallylamine, or a polypeptide.

4. The cell detachment agent according to claim 1 or 2, wherein the ureido group-containing polymer comprises a ureido group-containing repeating sequence A that is a repeat of a ureido group-containing structure represented by the following formula (2):

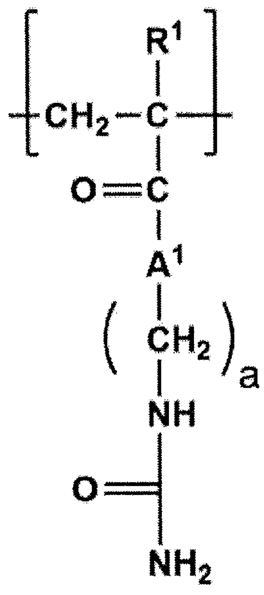

· · · (2)

in the formula (2), $R^1$ is a hydrogen atom or a methyl group, $A^1$ is O or NH, and a is an integer of 1 to 6.

5. The cell detachment agent according to claim 4, wherein the ureido group-containing polymer further comprises a hydrophilic repeating sequence B that is a repeat of a hydrophilic structure represented by the following formula (3):

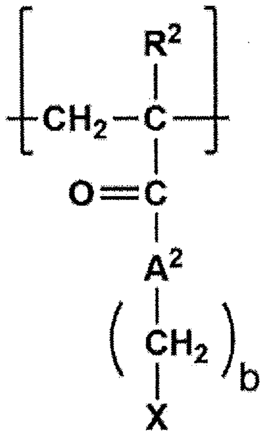 • • • (3)

in the formula (3), $R^2$ is a hydrogen atom or a methyl group, $A^2$ is O or NH, b is an integer of 1 to 6, and X is selected from the following formulas (4) to (9):

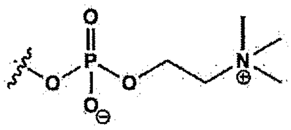 • • • (4) 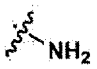 • • • (5) 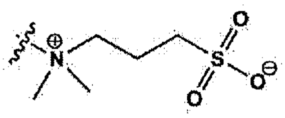 • • • (6)

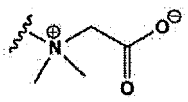 • • • (7) 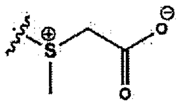 • • • (8) 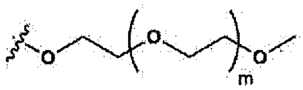 • • • (9)

in the formula (9), m is an integer of 0 to 20.

**6.** The cell detachment agent according to claim 5, wherein, in the ureido group-containing repeating sequence A, $A^1$ is O and a is 2, and in the hydrophilic repeating sequence B, $A^2$ is O, b is 2, and X is represented by the formula (4).

**7.** The cell detachment agent according to claim 5, wherein the ureido group-containing repeating sequence A and the hydrophilic repeating sequence B in the ureido group-containing polymer are in a relationship of $0.5 \leq A/(A+B)$ in a molar ratio.

**8.** The cell detachment agent according to claim 4, wherein the bindable ureido group-containing polymer has the biotin residue as the separation carrier-binding site at the terminal of the ureido group-containing polymer.

**9.** The cell detachment agent according to claim 1 or 2, wherein the ureido group-containing polymer comprises a ureido group-containing repeating sequence C that is a repeat of a ureido group-containing structure represented by the following formula (10) and a hydrophilic repeating sequence D that is a repeat of a hydrophilic structure represented by the following formula (11):

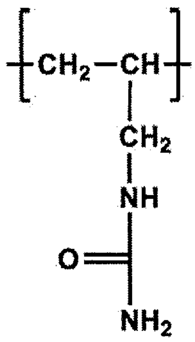

・・・(10)

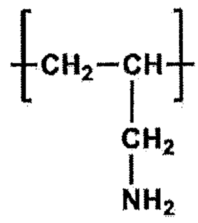

・・・(11).

**10.** The cell detachment agent according to claim 9, wherein the ureido group-containing repeating sequence C and the hydrophilic repeating sequence D in the ureido group-containing polymer are in a relationship of $0.5 \leq C/(C+D)$ in a molar ratio.

**11.** The cell detachment agent according to claim 9, wherein the agent comprises the bindable ureido group-containing polymer having the biotin residue as the separation carrier-binding site at the side chain of the ureido group-containing polymer.

**12.** The cell detachment agent according to claim 1 or 2, wherein the ureido group-containing polymer comprises a ureido group-containing repeating sequence E that is a repeat of a ureido group-containing structure represented by the following formula (12) and a hydrophilic repeating sequence F that is a repeat of a hydrophilic structure represented by the following formula (13):

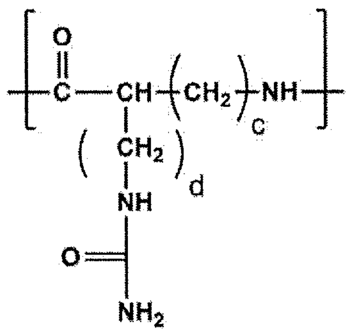

・・・(12)

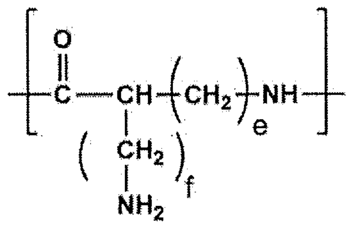

・・・(13)

in the formula (12), c is an integer of 0 to 6 and d is an integer of 0 to 6, and in the formula (13), e is an integer of 0 to 6 and f is an integer of 0 to 6.

**13.** The cell detachment agent according to claim 12, wherein, in the ureido group-containing repeating sequence E, c is 0 and d is 3, and in the hydrophilic repeating sequence F, e is 0 and f is 3.

**14.** The cell detachment agent according to claim 12, wherein the ureido group-containing repeating sequence E and the hydrophilic repeating sequence F in the ureido group-containing polymer are in a relationship of $0.5 \leq E/(E+F)$ in a molar ratio.

**15.** The cell detachment agent according to claim 12, wherein the agent comprises the bindable ureido group-containing polymer having the biotin residue as the separation carrier-binding site at the side chain of the ureido group-containing polymer.

**16.** The cell detachment agent according to claim 1 or 2, wherein the ureido group-containing polymer has a number average molecular weight of 20,000 to 1,000,000.

**17.** A method for separating a cell to be separated from a cell population comprising the cell to be separated, comprising

the following [step A], [step B], [step C], and [step D]:

[step A]
a step of adding a substance having a separation carrier-binding site and recognizing a surface antigen of the cell to be separated to a cell population comprising the cell to be separated to cause a reaction with the surface antigen of the cell to be separated, thus obtaining a complex of the cell to be separated-substance recognizing the antigen
[step B]
a step of interacting the complex of the cell to be separated-substance recognizing the antigen obtained in [step A] with a separation carrier via a separation carrier-binding site to obtain a complex of the cell to be separated-separation carrier
[step C]
a step of separating only the complex of the cell to be separated-separation carrier obtained in [step B] from the cell population comprising the cell to be separated
[step D]
a step of adding a cell detachment agent according to claim 1 or 2 to the complex of the cell to be separated-separation carrier obtained in [step C] and separating the cell to be separated by temperature change.

**18.** The cell separation method according to claim 17, wherein the separation carrier-binding site in the [step A] is a biotin residue, a streptavidin residue, an amino group, a carboxyl group, a mercapto group, a cyclodextrin residue, an adamantyl group, or a phenylboronic acid residue.

**19.** The cell separation method according to claim 17, wherein the substance recognizing the antigen in the [step A] is an antibody, an antibody fragment, a peptide, an aptamer, protein A, or lectin.

**20.** The cell separation method according to claim 17, wherein the separation carrier in the [step B] is a magnetic particle.

**21.** The cell separation method according to claim 20, wherein only the complex of the cell to be separated-separation carrier is separated by magnetic force from the cell population comprising the cell to be separated in the [step C].

**22.** The cell separation method according to claim 17, wherein the temperature change in the [step D] comprises heating from a temperature not more than the upper critical solution temperature (UCST) to a temperature not less than the upper critical solution temperature (UCST) of the bindable ureido group-containing polymer.

**INTERNATIONAL SEARCH REPORT**

International application No. **PCT/JP2022/025542**

**A. CLASSIFICATION OF SUBJECT MATTER**

***C12N 1/02***(2006.01)i; ***C12N 5/071***(2010.01)i; ***G01N 33/53***(2006.01)i; ***G01N 33/543***(2006.01)i
FI: C12N1/02; G01N33/543 581W; G01N33/53 U; C12N5/071; G01N33/543 581U

According to International Patent Classification (IPC) or to both national classification and IPC

**B. FIELDS SEARCHED**

Minimum documentation searched (classification system followed by classification symbols)

C12N1/02; C12N5/071; G01N33/53; G01N33/543

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| Y | JP 7-136505 A (TERUMO CORP) 30 May 1995 (1995-05-30) paragraphs [0010], [0012]-[0014], [0020], example 1 | 1-16 |
| Y | WO 2011/118587 A1 (KYUSHU UNIVERSITY) 29 September 2011 (2011-09-29) claims, paragraphs [0114], [0117] | 1-16 |
| Y | JP 2015-174962 A (TOKYO INST TECH) 05 October 2015 (2015-10-05) claims, paragraphs [0034], [0064], [0069] | 1-16 |
| Y | 畔柳　奏太郎　ほか, UCST型ウレイドポリマーの調製とバイオマテリアルへの展開, 高分子論文集, 20 April 2017, vol. 74, no. 4, pp. 250-256 p. 252, left column, fig. 4, (KUROYANAGI, Sotaro et al. Preparation and Biomaterial Application of UCST-Type Ureido Polymer. KOBUNSHI RONBUNSHU.) | 12-15 |

☐ Further documents are listed in the continuation of Box C.　　☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **05 September 2022** | **13 September 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2022/025542**

| Patent document cited in search report | Publication date (day/month/year) | Patent family member(s) | Publication date (day/month/year) |
|---|---|---|---|
| JP 7-136505 A | 30 May 1995 | (Family: none) | |
| WO 2011/118587 A1 | 29 September 2011 | US 2013/0012597 A1<br>claims, paragraphs [0096], [0099]<br>EP 2551285 A1 | |
| JP 2015-174962 A | 05 October 2015 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2016136937 A
- JP 2017514481 A
- JP 5800323 B
- WO 9931144 A
- WO 9801478 A
- US 6153705 A
- JP 2021109278 A